# EUROPEAN PATENT APPLICATION

(11) **EP 3 569 708 A1**
(43) Date of publication of application: **20.11.2019**
(21) Application number: 18738823.6
(22) Date of filing: 09.01.2018
(51) Int. Cl.: C12N 15/09, A61K 35/761, A61K 38/00, A61K 38/43, A61K 48/00, A61P 1/16, A61P 7/04

(54) **AAV VECTOR FOR DISRUPTING CLOTTING-RELATED FACTOR GENE ON LIVER GENOME**

(30) Priority: 13.01.2017 JP 2017004198
(71) Applicant: Jichi Medical University, Tokyo 102-0093 (JP)
(72) Inventor: OHMORI Tsukasa, Tokyo Tokyo 102-0093 (JP); NAGAO Yasumitsu, Tokyo Tokyo 102-0093 (JP); MIZUKAMI Hiroaki, Tokyo Tokyo 102-0093 (JP); SAKATA Asuka, Tokyo Tokyo 102-0093 (JP); OZAWA Keiya, Tokyo Tokyo 102-0093 (JP); MURAMATSU Shin-ichi, Tokyo Tokyo 102-0093 (JP); TOMINAGA Shin-ichi, Tokyo Tokyo 102-0093 (JP); HANAZONO Yutaka, Tokyo Tokyo 102-0093 (JP); NISHIMURA Satoshi, Tokyo Tokyo 102-0093 (JP); SAKATA Yoichi, Tokyo Tokyo 102-0093 (JP); KAMOSHITA Nobuhiko, Tokyo Tokyo 102-0093 (JP)
(74) Representative: Elkington and Fife LLP
(86) International application number: PCT/JP2018/000150
(87) International publication number: WO 2018/131551

(57) **Abstract**

The present invention provides a recombinant adeno-associated virus (rAAV) vector for treating a blood coagulation-related disease to provide a novel gene therapy means for hemophilia. The virus vector comprises a virus genome comprising a liver-specific promoter sequence and a polynucleotide sequence encoding a genome editing means operably linked to the promoter sequence, wherein the genome editing means is (a) a means comprising CRISPR/Cas9 composed of a Cas9 protein and a guide RNA (gRNA) and a repair gene, or (b) a means comprising CRISPR/Cas9 composed of a Cas9 protein and a gRNA, and the gRNA comprises a nucleotide region complementary to a part of a region related to expression of a disease-related protein on the genome of a patient and a region that interacts with the Cas9 protein.

## Description

### [Technical Field]

The present invention relates to a recombinant adeno-associated virus vector (rAAV) for treating a blood coagulation factor (clotting factor) disease using a genome editing technique. More specifically, the present invention relates to rAAV comprising a liver-specific promoter and a genome editing means, optionally combining the AAV with another rAAV comprising a repair gene, a pharmaceutical composition for treating a blood coagulation disease comprising the aforementioned rAAV, and the like.

### [Background Art]

Hemophilia is a congenital disease with which the blood does not clot properly, and hemophilia A and B are known. Those with hemophilia A and B are genetically deficient in blood coagulation factor VIII and blood coagulation factor XI, respectively. The numbers of patients with hemophilia A and hemophilia B are respectively estimated to be 400000 or more and 80000 to 100000 in the whole world. According to Hemophilia Topics (vol.23, published in January 2011), the number of patients with hemophilia in Japan is approximately 8.5 per 100000 males and the numbers of patients with hemophilia A and hemophilia B reported in 2009 are 4317 and 933, respectively. In the world, the incidence rates in U.K., Netherlands, Sweden, Canada, and Germany, which are 19.1, 17.2, 15.3, 13.2, and 10.4 per 100000 males respectively, and the like are higher than that in Japan. The incidence rate in U.S.A. is 7.8 per 100000 males and approximately the same as that in Japan.

In conventional techniques of gene therapies for hemophilia, the genomic abnormality that the patient has is usually left as it is and a normal gene (for example, cDNA of a normal blood coagulation factor VIII and a promoter for its expression, and the like) is separately introduced (Patent Literature 1, Patent Literature 2). In such techniques, gene therapies targeting hepatocytes using an adeno-associated virus (AAV) vector have shown clear treatment results in clinical studies (Non Patent Literature 1).

Introduction of a recombinant AAV vector into the living body may induce a neutralizing antibody against the AAV vector in the living body and make it unlikely to have subsequent positive therapeutic effect. The possession rate of such a neutralizing antibody is approximately 40% and higher in adults. Therefore, childhood is suitable for a gene therapy with an AAV vector. However, even if gene expression is by AAV, the effect of a gene therapy is actually often lost over time in childhood, when hepatocytes proliferate. Therefore, if a gene therapy that can target any genomic site in a patient becomes available, more patients are expected to become suitable for the therapy.

For the genome editing technique that targets and cuts a particular genomic site, proteins and protein complexes such as ZFN, TALEN, and CRISPR/Cas9 have attracted attention (Patent Literature 3, Patent Literature 4, Non-Patent Literature 2 to 4). In particular, CRISPR-Cas9 has a simpler structure, high flexibility in sequence designing, and also good success rates of genome editing, and is of low cost and therefore its application is rapidly widening (Non Patent Literature 4).

Although there has been a report of the Cas9 expression using AAV, it aims for cholesterol control, but not for treatment of a hemorrhagic disease such as hemophilia (Non Patent Literature 5). Moreover, gene editing techniques for the purpose of treating hemophilia have been reported, but the treatments use a genome editing technique other than the Cas9 (Patent Literature 6, Patent Literature 7).

### [Citation List]

### [Patent Literature]

[Patent Literature 1] JP-A-2015-509711
[Patent Literature 2] JP-A-2016-525356
[Patent Literature 3] U.S. Patent No. 8697359
[Patent Literature 4] JP-A-2015-510778
[Patent Literature 5] JP-A-2016 -524472
[Patent Literature 6] WO 2015/089046
[Patent Literature 7] WO 2014/089541

### [Non Patent Literature]

[Non Patent Literature 1] J Thrombosis and Haemostasis. 2015; 13: S133-S142.; Mol Ther. 2013; 21:318-323
[Non Patent Literature 2] Li, H. et. al., (2011) Nature 475, 217-221
[Non Patent Literature 3] Shalem, O et. al., (2015) Nature 520, 186-191
[Non Patent Literature 4] Ran, F.A. et. al., (2013) Cell 154, 1380-1389

### [Summary of Invention]

### [Technical Problem]

Therefore, the development of therapeutic recombinant virus vector for treatment of a genetic disease related to genome damage in hepatocytes, for example, hemophilia, thrombophilia, thrombocytopenia, hereditary hemorrhagic telangiectasia, hereditary liver metabolic abnormalities, hepatocellular carcinoma, or the like, having higher therapeutic effect, and in combination with a genome editing technique is demanded.

### [Solution to Problem]

The present invention relates to an rAAV vector that expresses Cas9 with a liver-specific promoter such as a combined promoter of ApoE enhancer and antitrypsin promoter and has a guide RNA sequence to an anticoagulant factor, such as antithrombin, or a coagulation factor, with U6 promoter, a pharmaceutical composition that disrupts a candidate gene in hepatocytes using the vector *in vivo* by the technique and ameliorates the bleeding tendency in a hemorrhagic disease such as hemophilia, and the like.

Accordingly, the present invention provides the following inventions, such as an rAAV vector for gene therapy of hemophilia, a pharmaceutical composition comprising the rAAV vector.
[1] A recombinant adeno-associated virus (rAAV) vector for treatment of a blood coagulation factor disease,
   wherein the virus vector comprises a viral genome comprising a liver-specific promoter sequence and a polynucleotide sequence encoding a genome editing means operably linked to the promoter sequence,
   said genome editing means is
   (a) a means comprising CRISPR/Cas9 composed of a Cas9 protein and a guide RNA (gRNA) and a repair gene, or
   (b) a means comprising CRISPR/Cas9 composed of a Cas9 protein and a gRNA,
   wherein the gRNA comprises a nucleotide region complementary to a part of a region related to expression of a disease-related protein on the genome of a patient and a region that interacts with the Cas9 protein.
[2] The rAAV vector according to [1], wherein the vector comprises a capsid protein derived from an adeno-associated virus of AAV3, AAV3B, AAV8, or AAV9.
[3] The rAAV vector according to [1] or [2], wherein the liver-specific promoter sequence comprises a polynucleotide having 90% or more homology with a polynucleotide sequence selected from the group consisting of ApoE promoter, anti-trypsin promoter, cKit promoter, a promoter of a liver-specific transcription factor (HNF-1, HNF-2, HNF-3, HNF-6, C/ERP, or DBP), a promoter of albumin or thyroxine binding globulin (TBG), and the polynucleotide sequence set forth in SEQ ID NO: 1, and functions as a liver-specific promoter.
[4] The rAAV vector according to any one of [1] to [3], wherein the Cas9 protein comprises an amino acid sequence set forth in SEQ ID NO: 2 or 6 or comprises an amino acid sequence having 90% or more homology with an amino acid sequence set forth in SEQ ID NO: 2 or 6, and can be combined with a gRNA having a sequence of any one of SEQ ID NOs: 3 to 5 or SEQ ID NOs: 7 to 15 to form a CRISPR/Cas9 complex.
[5] The rAAV vector according to any one of claims [1] to [4], wherein the gRNA has a polynucleotide sequence set forth in any of SEQ ID NOs: 3 to 5 or SEQ ID NOs: 7 to 15, or comprises a polynucleotide sequence having 90% or more homology with a polynucleotide sequence set forth in any of SEQ ID NOs: 3 to 5 or SEQ ID NOs: 7 to 15 (3' sequence without crRNA and PAM portions), and can be combined with a Cas9 protein having a sequence set forth in SEQ ID NO: 2 or 6 to form a CRISPR/Cas9 complex.
[6] The reAAV vector according to any one of claims [1] to [5], wherein the disease-related protein is a blood coagulation factor or an anticoagulant factor.
[7] The rAAV vector according to [6], wherein the disease-related protein is blood coagulation factor IX, antitrypsin, blood coagulation factor VIII, blood coagulation factor VII, blood coagulation factor XI, antithrombin, protein S, or protein C.
[8] The RAAV vector according to any one of [1] to [7], wherein the (a) comprises (a1) an adeno-associated virus vector encoding the CRISPR/Cas9 composed of the Cas9 protein and the gRNA and (a2) an adeno-associated virus vector comprising the repair gene.
[9] The RAAV vector according to [8], wherein the (a2) comprises regions homologous to 5' and 3' sides of the cleavage target site in the genome on the 5' and 3' sides of the repair gene.
[10] The rAAV vector according to [9], wherein the repair gene comprises a polynucleotide encoding a normal disease-related protein or a part thereof between the regions homologous to the 5' and 3' sides.
[11] The rAAV vector according to any one of [1] to [10], wherein the blood coagulation-related disease is selected from the group consisting of hemophilia, factor VII deficiency, factor XI deficiency, antithrombin deficiency, protein S abnormality/deficiency, and protein C abnormality.
[12] The rAAV vector according to any one of [1] to [11], wherein the virus genome further comprises a nucleotide sequence of an inverted terminal repeat (ITR) selected from the group consisting of AAV1, AAV2, AAV3, AAV4, AAV6, and AAV9.
[13] A pharmaceutical composition for treating a liver-related disease, comprising the rAAV vector according to any of [1] to [12].
[14] The pharmaceutical composition according to [13], wherein the pharmaceutical composition comprises a combination of two or more rAAV vectors.
[15] The pharmaceutical composition according to [13] or [14], wherein the pharmaceutical composition is used in combination with a therapeutic agent for hemophilia.
[16] A medical kit for treating a liver-related disease, comprising a pharmaceutical composition according to any of [13] to [15].
[17] A method of using the vector according to any one of [1] to [12], for treatment of hemophilia, liver metabolic abnormality, liver cell cancer, or the like.
[18] A method of treatment, comprising administering the vector according to any one of [1] to [12] to a patient.
[19] The method of treatment according to [18], wherein the vector according to any one of [1] to [12] comprises two or more vectors and the method comprises administering the two or more vectors simultaneously.

### [Advantageous Effects of Invention]

By using the virus vector according to the present invention, a therapeutic recombinant virus vector combined with a genome editing technique and having higher therapeutic effect can be provided that is available in treatment of a genetic disease relating to genome damage in hepatocytes, for example, hemophilia, thrombophilia, thrombocytopenia, hereditary hemorrhagic telangiectasia, hereditary liver metabolic abnormality, hepatocellular carcinoma, or the like. Moreover, by using the vector according to the present invention, it becomes allowing to make genome editing in most hepatocytes.

### [Brief Description of the Drawings]

[FIG. 1] FIGs. 1A to IE show that hemophilia B mice were generated by injection of sgRNA and SpCas9 mRNA into zygotes.
[FIG. 2] FIGs. 2A to 2D show AAV vector-mediated generation of hemophilia B in adult wild-type mice.
[FIG. 3] FIGs. 3A to 3C show marginal increase in FIX:C by HDR.
[FIG. 4] FIG. 4 is a schematic diagram of targeting strategy.
[FIG. 5] FIGs. 5A to 5F show increases in plasma FIX:C in hemophilia B mice by CRIPSR/Ca9-mediated genome editing using an AAV8 vector.
[FIG. 6] FIGs. 6A to 6F show the rescue of the bleeding phenotype of hemophilia B mice by disruption of the AT gene, Serpinc1.
[FIG. 7] FIGs. 7A and 7B show the results of comparison between an HCRhAAT promoter and a TBG promoter in mice.

### [Description of Embodiments]

The present invention allows to treat a genetic disease relating to genome damage in hepatocytes, particularly a hemorrhagic disease caused by genetic mutation in hepatocytes, and more specifically hemophilia, by using a genome editing technique.

### 1. Hemophilia and Relative Factors

Hemophilia is a representative congenital hemorrhagic disease and patients with hemophilia repeat hemorrhage from their childhood. There are 2 types of hemophilia: hemophilia A and hemophilia B. Hemophilia A is caused by deficiency and/or abnormality in blood coagulation factor VIII (also referred to as FVIII) and hemophilia B is caused by deficiency and/or abnormality in blood coagulation factor IX (also referred to as FIX). Both Hemophilia A and hemophilia B are not distinguishable from each other based on indicated symptoms, and thus their identification requires genetic diagnosis. Since both of the causative genes of hemophilia locate on the X chromosome and are of recessive genotypes, most patients are male. Therapies commonly performed are drug therapies by supplementation of plasma-derived or recombinantly-produced FVIII or FIX.

Blood coagulation factor VIII is a high molecular weight glycoprotein with a molecular weight of about 330 kDa (2351 amino acid residues) and produced mainly in the liver. The plasma concentration of FVIII is 0.01 to 0.02 mg/dL in healthy humans. Blood coagulation factor IX is a protein with a molecular weight of about 55 kDa (415 amino acid residues) and produced in the liver hepatocytes. The plasma concentration of FIX is 3 to 5 mg/dL in healthy humans. Therefore, FIX, in comparison with FVIII, has therapeutic advantages in that the target organ is limited to the liver and the size of the coding gene is smaller.

In the process of blood coagulation, thrombin plays an important role mediated via activation by blood coagulation factor V, activating blood coagulation factor VIII, or the like. Meanwhile, antithrombin (antithrombin III) forms one-to-one complexes with thrombin, active factor IX, active X, and the like in order to provide inhibition of blood coagulation (or anticoagulation). For example, in patients who have a reduced level of a coagulation factor and/or in a case when patients have an insufficient activity of a coagulation factor, the blood coagulation can be promoted by supplementing the coagulation factor. Alternatively, reduction or deletion of the activity of the antithrombin, which inhibits the coagulation, may promote the blood coagulation.

The present invention provides a virus vector that restores the blood coagulation by using particular genome editing means, preferably in patients having congenital factor deficiency in a blood coagulation, and a pharmaceutical composition comprising the virus vector. Possible examples of the means for allowing to promote the blood coagulation can include (a) a means for repairing a genome comprising a blood coagulation factor with reduced function or deficiency or (b) a means for restoring blood coagulation by inhibiting anticoagulation in a patient with reduced blood coagulation action.

### 2. Recombinant virus vector

### 2.1. Recombinant adeno-associated virus (rAAV) vector used in present invention

In the present invention, as a vector for delivering a polynucleotide for a genome editing means, or a polynucleotide encoding a particular blood coagulation factor to an organ of a subject, an rAAV vector capable of delivering a gene in an amount sufficient for exhibiting a therapeutic effect even in peripheral administration, as described in WO2012/057363, or such a vector as described in WO2008/124724, etc. can be used.

Naturally-occurring adeno-associated viruses (AAVs) are nonpathogenic viruses. Based on said feature, various recombinant virus vectors have been prepared and used in order to deliver a desired gene for gene therapy (see, for example, WO2003/018821; WO2003/053476; WO2007/001010; and Journal of the Pharmaceutical Society of Japan (Yakugakkai-shi) 126 (11) 1021-1028). The wild-type AAV genome is a single-stranded DNA molecule which has a nucleotide length of approximately 5 kb in the full length, and is sense or antisense. In general, the AAV genome has invert terminal repeat (ITR) sequences of approximately 145 nucleotides long at both the 5' and 3' ends of the genome. It has been known that the ITR possesses diverse functions such as a function as a replication origin of the AAV genome and a function as a packaging signal for this genome into a virion (see, for example, the above-described literature: Journal of the Pharmaceutical Society of Japan (Yakugakkai-shi) 126 (11) 1021-1028). A region flanked by the ITR sequences in the wild-type AAV genome (hereinafter, referred to as an internal region) contains an AAV replication (rep) gene and a capsid (cap) gene. The rep gene and the cap gene encode Rep, a protein involved in the virus replication, and a capsid protein constituting an outer coat capsomere having an icosahedral structure (e.g., at least one of VP1, VP2 and VP3), respectively. For more details, see, for example, Human Gene Therapy, 13, pp. 345-354, 2002; Neuronal Development 45, pp. 92-103, 2001; Experimental Medicine 20, pp. 1296-1300, 2002; Journal of the Pharmaceutical Society of Japan 126 (11) 1021-1028; and Hum Gene Ther, 16, 541-550, 2005.

The rAAV vector of the present invention is preferably a vector derived from naturally-occurring adeno-associated virus type 1 (AAV1), type 2 (AAV2), type 3 (AAV3), type 4 (AAV4), type 5 (AAV5), type 6 (AAV6), type 7 (AAV7), type 8 (AAV8), type 9 (AAV9), or type 10 (AAV10) of origin, but not limited thereto. The nucleotide sequences of the genomes of these adeno-associated viruses have been known in the art, and can be in reference to the nucleotide sequences of GenBank registration Nos: AF063497.1 (AAV1), AF043303 (AAV2), NC_001729 (AAV3), NC_001829.1 (AAV4), NC_006152.1 (AAV5), AF028704.1 (AAV6), NC_006260.1 (AAV7), NC_006261.1 (AAV8), and AY530579 (AAV9). In the present invention, it is preferred, particularly for direction to hepatocyte, to adopt a capsid protein (VP1, VP2, VP3, etc.) derived from AAV3, AAV3B (AF028705.1), AAV8, or AAV9. The amino acid sequences of these capsid proteins have been known in the art. See, for example, the amino sequence registered in the corresponding GenBank registration No. of each AAV.

The Rep protein used in the present invention is used for replicating the rAAV of the present invention. Such a Rep protein may contain deletion, substitution, insertion and/or addition of amino acids of preferably 20 or less residues, 15 or less residues, 10 or less residues, or 5 or less residues, as long as the Rep protein possesses a function of recognizing the ITR sequences and performing genome replication dependent on the sequences, a function of packaging the wild-type AAV genome (or rAAV genome) into a viral virion, and/or a function of forming the virion of the rAAV of the present invention, in order to replicate the rAAV of the present invention, at the same level as the function(s) of the wild type. In this context, the same level as the function(s) in the wild type means that specific activity (activities) is 50%, 60%, 70%, 80%, or 90% or more based on the activity (activities) of the wild type. In the present invention, preferably, an AAV3-derived Rep protein known in the art is used, but not limited thereto.

In one embodiment of the present invention, the capsid protein VP1 or the like (VP1, VP2 and/or VP3) and the Rep protein encoded in the internal region of the wild-type AAV genome described above are used for obtaining the rAAV of the present invention by inserting polynucleotides encoding these proteins into an AAV helper plasmid. The capsid protein (VP1, VP2 and/or VP3) and the Rep protein used in the present invention may be inserted, if necessary, in one, two, or three or more plasmids. Optionally, one or more of the capsid protein and the Rep protein may be contained in the AAV genome. In the present invention, preferably, both the capsid protein (VP1, VP2 and/or VP3) and the Rep protein are encoded by one polynucleotide and provided as an AAV helper plasmid.

### 2.2. Polynucleotide contained in rAAV of present invention

### (1) rAAV virus genome

The polynucleotide to be packaged in the rAAV vector of the present invention (i.e., the polynucleotide) can be prepared by replacing the polynucleotide of the internal region (i.e., one or both of the rep gene and the cap gene) flanked by the ITR sequences positioned on the 5' and 3' sides of the wild-type genome, with a gene cassette comprising, for example, a polynucleotide encoding the protein of interest (a genome editing means and/or a repair gene) and a promoter sequence for transcribing this polynucleotide. Preferably, the ITR sequences positioned on the 5' and 3' sides are positioned at the 5' and 3' ends of the AAV genome, respectively. Preferably, in the rAAV genome of the present invention, the ITR sequences positioned at the 5' and 3' ends include 5' ITR and 3' ITR contained in the genome of AAV1, AAV2, AAV3, AAV6 or AAV9, but not limited to these particular AAVs.

In general, the ITR portion may easily form a sequence replaced with a complementary sequence (flip and flop structure). Therefore, the 5' and 3' directions of the ITR sequences contained in the rAAV genome of the present invention may be inverted. In the rAAV genome of the present invention, the length of the polynucleotide replaced with the internal region (i.e., a genome editing means and/or a repair gene) is practically preferably almost the same as the length of the original polynucleotide. Specifically, the full length of the rAAV genome of the present invention is preferably almost the same as 5 kb, which is the full length of the wild type, and is, for example, approximately 2 to 6 kb, and preferably approximately 4 to 6 kb.

The length of the therapeutic gene to be integrated in the rAAV genome of the present invention other than the length of a transcriptional regulatory region including a promoter, polyadenylation and the like (e.g., assuming that the length is approximately 1 to 1.5 kb) preferably correspond to a length of approximately 0.01 to 3.7 kb, more preferably a length of approximately 0.01 to 2.5 kb, and further preferably approximately 0.01 to 2 kb, but not limited thereto.

A plurality (two or more types) of therapeutic genes may be co-integrated in the rAAV genome by use of a means known in the art, such as the intervention of an internal ribosome entry site (IRES) sequence, as long as the full length of the rAAV genome falls within the range as described above. When the rAAV of the present invention expresses a plurality (two or more) of proteins, respective genes encoding these proteins may be located in the same direction or in different directions.

In general, a single-stranded polynucleotide packaged in a rAAV virion may require a time (several days) for expressing the genome editing means and/or the repair gene of interest. In this case, the genome editing means and/or the repair gene to be introduced may be designed so as to be of sc (self-complementary) type having self-complementarity so that the effect of the rAAV is exerted in a shorter time. Details are specifically described in, for example, Foust K.D. et al. (Nat Biotechnol. 2009 Jan; 27 (1): 59-65). The polynucleotide packaged in the rAAV vector of the present invention may be of non-sc type or sc type. Preferably, the polynucleotide packaged in the rAAV vector of the present invention is of non-sc-type.

### (2) Promoter sequence used in present invention

In one embodiment, the rAAV vector of the present invention preferably comprises a polynucleotide comprising a hepatocyte-specific promoter sequence and a genome editing means and/or a repair gene operably linked to the promoter sequence (i.e., such a polynucleotide is packaged in the rAAV vector of the present invention). The promoter sequence used in the present invention can be a promoter sequence specific to the cells contained in the liver, for example, a promoter sequence specific for hepatic parenchymal cells, hepatic non-parenchymal cells (astrocytes, etc.), or the like. Examples of such a promoter sequence specifically include, but are not limited to, ApoE promoter, anti-trypsin promoter, cKit promoter, promoters of liver-specific transcription factors (HNF-1, HNF-2, HNF-3, HNF-6, C/ERP, DBP, etc.), thyroxine binding globulin (TBG) promoter (Nature. 2015 Apr 9; 520 (7546): 186-91) (SEQ ID NO: 34), a human SEPP1 promoter region (liver-specific promoter) (SEQ ID NO: 35), promoters of other liver-specific proteins (albumin, etc.), and synthetic promoters from these promoters in combination. Also, these promoters can be further combined with an enhancer sequence known in the art, preferably a hepatocyte-specific enhancer sequence. Examples of the enhancer sequence that can be used in the present invention include an ApoE enhancer sequence and human TIE2 enhancer (endothelial cell-specific enhancer) (SEQ ID NO: 36). One each of or any combination of two or more each of these promoter sequences and enhancer sequences may be used. Furthermore, a synthetic promoter may be used through the use of the hepatocyte-specific promoter and enhancer described above. Examples of such a synthetic promoter/enhancer sequence include, but are not limited to, HCRhAAT synthetic promoter (SEQ ID NO: 1). The rAAV vector of the present invention may comprise preferably a liver specific promoter, and more preferably a promoter specific to hepatic parenchymal cells (hepatocytes), including a sequence of ApoE promoter, anti-trypsin promoter, TBG promoter, or HCRhAAT synthetic promoter.

Alternatively, the hepatocyte-specific promoter sequence used in the present invention may be a polynucleotide having the deletion, substitution, insertion and/or addition of one or more (e.g., 1 to 100, 1 to 50, 1 to 40, 1 to 30, 1 to 25, 1 to 20, 1 to 15, 1 to 10, 1 to 9 (1 to several), 1 to 8, 1 to 7, 1 to 6, 1 to 5, 1 to 4, 1 to 3, 1 or 2, or 1) nucleotides in the polynucleotide sequence of the promoter described above, and capable of functioning as a hepatocyte-specific promoter sequence. In the present specification, the capability of functioning as a hepatocyte-specific promoter sequence means that, for example, when comparison between liver-derived cells with non-liver-derived cells are performed in a reporter assay, the non-liver-derived cells express a reporter at a detection threshold, or express approximately 25% or less, approximately 20% or less, approximately 15% or less, or approximately 10% or less of a reporter expressed by the liver-derived cells. The above-described polynucleotide having the deletion, substitution, insertion and/or addition means that its specific activity based on the original promoter sequence is 50%, 60%, 70%, 80%, or 90% or more. A smaller number of mutations described above is more preferred.

In the rAAV vector of the present invention, a promoter known in the art, such as a U6 promoter sequence, a U5 promoter sequence, or a U4 promoter sequence can be used, particularly, for suitable expression of guide RNA. Optionally, promoter sequences known and commonly-used in the art, such as CMV promoter or CAG promoter may be used alone or in combination with any of other promoters described above. In a further embodiment, only one promoter may be used if IRES is used.

### (3) Therapeutic polynucleotide used in present invention

While a virus genome contained in an AAV vector is usually incorporated into the host chromosome, it may be necessary to integrate the foreign gene into a particular position in the genome in the cells, for the purpose of more stable continued existence of a newly incorporated foreign gene in cells.

It had not been previously easy to perform gene recombination at a particular site in the genomes of living cells differentiated into each organ or others. However, use of recently developed means capable of cleaving a particular genome position (genome editing means) has enabled double-strand break (DSB) targeting the particular genome position (Patent Literature 3, etc.). Genomic DNA that has undergone intracellular double-strand break (DSB) is usually repaired by any of non-homologous end-joining repair and homologous recombination repair.

The non-homologous end-joining repair is a mechanism that is exploited in the absence of proper repair template DNA, and refers to the repair of cleaved ends. This repair is known to easily cause errors, and specifically, the insertion of several bases or the deletion of several bases occurs at random. When such random repair occurs in a protein coding region, the coding region may be disrupted or dysfunctional due to frameshift, resulting in reduced expression of a disadvantageously functioning gene.

Alternatively, the repair template DNA which is used for the repair of the genome having double-strand break has regions homologous to the upstream and downstream sides of the cleavage target site in the genome. When the normal gene or gene portion (repair gene) of interest is placed between these regions, the genome having double-strand break is repaired via homologous recombination, resulting in enabling replacement of the target site with the normal gene or gene portion (homologous recombination repair). Furthermore, an additional expression cassette may be integrated therein. This homologous recombination repair is capable of providing a repair with higher accuracy, in contrast to a random repair as described above.

The above-described genome editing means using genomic frameshift and/or repair enables the treatment of a genetic disease. Particularly, cells that have undergone genomic editing are then capable of proliferation bearing the edited genome and may therefore bring about great benefits, for example, to child patients having active cell division.

### (4) Genome editing means according to present invention

The virus vector of the present invention may comprise a polynucleotide for expressing CRISPR/Cas9 which is a means capable of specifically cleaving a target site in the genome, as well as a liver-specific promoter sequence, as the genome editing means. As used herein, the genome editing means refers to involvement of a genomic cleavage means alone, or the genomic cleavage means together with a genomic repair means. The genome editing means is delivered to the liver through the use of the virus vector of the present invention so that specific genomic editing occurs in hepatocytes. More preferably, the virus vector of the present invention possesses higher direction and/or expression specificity to the liver.

Means for specifically cleaving a target site in the genome include those known in the art using a protein-nucleic acid complex, such as ZFN, TALEN, or CRISPR/Cas9 (Patent Literature 3, Patent Literature 4, Non Patent Literatures 2 to 5, etc.). Among others, one molecule of each of ZFN and TALEN cleaves one genome strand, and two types of recombinant ZFN or TALEN molecules are therefore required for editing double-stranded genomic DNA. On the other hand, one molecule of CRISPR/Cas9 can cleave two genome strands and is therefore capable of editing genomic DNA by use of one type of recombinant CRISPR/Cas9. Thus, the present invention is preferably the virus vector which expresses a recombinant CRISPR/Cas9 as the genome editing means.

In the case of performing a non-homologous end-joining repair which targets a coding region of a protein of interest, exon 1, exon 2 or the like in the coding region may be designed as a cleavage target. This repair introduces, for example, a random mutation into a portion to be repaired, so that a dysfunctional mutant protein can be expressed, or a target gene product can be no longer expressed.

In the case of designing a repair template for adopting a homologous recombination repair, it may be necessary to allow a PAM sequence to be absent immediately after a target sequence, or to remove or mutate a PAM sequence by using codon substitution or the like.

For a specific approach such as a repair mechanism or a target design method as to the genomic editing technology, see, for example, a review such as Curr Issues Mol Biol. 2016; 20: 1-12., and online software and its manual provided by Thermo Fisher Scientific Inc. (Waltham, MA, USA) or Benchling (https://benchling.com).

CRISPR/Cas9 known in the art is composed of two components: (1) a Cas9 protein having endonuclease activity, and (2) a small guide RNA (sgRNA or gRNA). As used herein, the CRISPR/Cas9 (or CRISPR/Cas9 complex) refers to a complex of the Cas9 protein and the sgRNA, unless otherwise specified. In the present invention, preferably, the Cas9 protein (Cas9 enzyme) derived from the CRISPR (clustered regularly interspaced short palindromic repeat) gene locus of *Streptococcus pyogenes* type II, or the Cas9 protein derived from *Staphylococcus aureus* is used as (1) the Cas9 protein having the endonuclease activity. Alternatively, a Cas9 protein which is derived from any of other microbial species, and known in the art to participate in site-directed double-strand genomic break, for example, SpCas9, SaCas9, Cpf1, or St1Cas9, may be used. In the present invention, the Cas9 protein of CRISPR/Cas9 (SEQ ID NO: 2), SaCas9 (SEQ ID NO: 6) or Cpfl is preferably used.

In the present invention, (2) the small guide RNA (sgRNA) which is combined with the endonuclease (1) is preferably designed to be a single-stranded chimeric RNA and to contain bacterial tracrRNA and crRNA portions. Among them, the crRNA is a polynucleotide of approximately 20 bases positioned on the 5'-terminal side of the chimeric RNA. This polynucleotide exerts a homing function to a cleavage target in the genome and forms an RNA-DNA complex at a specific genomic DNA target site positioned immediately before a protospacer adjacent motif (PAM), thereby fixing the Cas9/gRNA complex on the target genome. Examples of the sequence of PAM include 5'-NGG. Specifically, an upstream 20-base genomic region to the NGG site is designed so as to be complementary to the sequence of the crRNA portion, and the Cas9 enzyme bound with the resultant sgRNA can provide genomic cleavage to the PAM of the target.

The Cas9 protein used in the present invention includes, for example, a protein that comprises an amino acid sequence having the deletion, substitution, insertion and/or addition of one or more amino acids in the amino acid sequence of the Cas9 protein (e.g., SEQ ID NO: 2 or 6), and can function as a CRISPR/Cas9 complex together with the original gRNA under physiological conditions. Two or more of the above-described deletion, substitution, insertion and addition of amino acids may occur at the same time. Examples of such a protein include a protein that comprises an amino acid sequence having the deletion, substitution, insertion and/or addition of, for example, 1 to 50, 1 to 40, 1 to 39, 1 to 38, 1 to 37, 1 to 36, 1 to 35, 1 to 34, 1 to 33, 1 to 32, 1 to 31, 1 to 30, 1 to 29, 1 to 28, 1 to 27, 1 to 26, 1 to 25, 1 to 24, 1 to 23, 1 to 22, 1 to 21, 1 to 20, 1 to 19, 1 to 18, 1 to 17, 1 to 16, 1 to 15, 1 to 14, 1 to 13, 1 to 12, 1 to 11, 1 to 10, 1 to 9 (1 to several), 1 to 8, 1 to 7, 1 to 6, 1 to 5, 1 to 4, 1 to 3, 1 or 2, or 1 amino acid residues in the amino acid sequence of SEQ ID NO: 2 or 6, and functions as a Cas9 enzyme under physiological conditions. A smaller number of amino acid residues to be deleted, substituted, inserted and/or added as described above is generally more preferred. CRISPR/Cas9 comprising the Cas9 protein having the mutation described above preferably shows 50%, 60%, 70%, 80%, or 90% or more activity in terms of specific activity based on the original CRISPR/Cas9.

The Cas9 protein used in the present invention includes a protein that has the amino acid sequence having identity to the amino acid sequence of the SEQ ID NO: 2 or 6, or an amino acid sequence having approximately 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity to the amino acid sequence of SEQ ID NO: 2 or 6 and has a double-strand DNA break activity at the same level as in the original CRISPR/Cas9 when forming CRISPR/Cas9 under physiological conditions. A larger numeric value of the identity is more preferred. Preferably, CRISPR/Cas9 comprising such a Cas9 protein has 50%, 60%, 70%, 80%, or 90% or more activity in terms of specific activity based on the original CRISPR/Cas9.

Examples of mutually substitutable amino acid residues for the protein (polypeptide) of the present invention are given below. Amino acid residues falling within the same group are mutually substitutable.
Group A: leucine, isoleucine, norleucine, valine, norvaline, alanine, 2-aminobutanoic acid, methionine, o-methylserine, t-butylglycine, t-butylalanine, and cyclohexylalanine;
Group B: aspartic acid, glutamic acid, isoaspartic acid, isoglutamic acid, 2-aminoadipic acid, and 2-aminosuberic acid;
Group C: asparagine and glutamine;
Group D: lysine, arginine, ornithine, 2,4-diaminobutanoic acid, and 2,3-diaminopropionic acid;
Group E: proline, 3-hydroxyproline, and 4-hydroxyproline;
Group F: serine, threonine, and homoserine; and
Group G: phenylalanine and tyrosine.

A protein substituted with an amino acid residue or residues can be prepared according to a method known to those skilled in the art, such as common genetically engineering techniques. As to such genetically engineering techniques, see, for example, Molecular Cloning 3rd Edition, J. Sambrook et al., Cold Spring Harbor Lab. Press. 2001, and Current Protocols in Molecular Biology, John Wiley & Sons 1987-1997.

Alternatively, the Cas9 protein used in the present invention may be encoded by, for example, a polynucleotide appropriately engineered on the basis of the codon preference of host cells. The polynucleotide encoding the preferred Cas9 protein used in the present invention includes, for example, a polynucleotide having the deletion, substitution, insertion and/or addition of one or more (e.g., 1 to 50, 1 to 40, 1 to 30, 1 to 25, 1 to 20, 1 to 15, 1 to 10, 1 to 9 (1 to several), 1 to 8, 1 to 7, 1 to 6, 1 to 5, 1 to 4, 1 to 3, 1 or 2, or 1) nucleotides in a polynucleotide sequence encoding the amino acid sequence of SEQ ID NO: 2 or 6, and encoding a protein comprising the amino acid sequence of SEQ ID NO: 2 or 6, or a protein that comprises an amino acid sequence having deletion, substitution, insertion and addition of one or more amino acids in the amino acid sequence of SEQ ID NO: 2 or 6, as described above, and functions as a Cas9 protein. Two or more of the above-described deletion, substitution, insertion and/or addition may occur in combination at the same time. A smaller number of nucleotides to be deleted, substituted, inserted and/or added as described above is generally more preferred. In the invention of the present application, the polynucleotide preferably includes, for example, a polynucleotide capable of hybridizing under stringent conditions to the polynucleotide encoding the amino acid sequence of SEQ ID NO: 2 or 6 or a complementary sequence thereof, the polynucleotide encoding a protein comprising the amino acid sequence of SEQ ID NO: 2 or 6, or a Cas9 protein comprising an amino acid sequence having deletion, substitution, insertion and/or addition of one or more amino acids in the amino acid sequence of SEQ ID NO: 2 or 6, as described above.

The hybridization can be performed according to a method known in the art or a method equivalent thereto, for example, a method described in Molecular Cloning 3rd Edition, J. Sambrook et al., Cold Spring Harbor Lab. Press. 2001. In the case of using a commercially available library, the hybridization can be performed according to a method described in an instruction manual provided by a manufacturer, etc. In this context, the "stringent conditions" may be any of low stringent conditions, moderately stringent conditions and highly stringent conditions. The "low stringent conditions" are conditions involving, for example, 5 × SSC, 5 × Denhardt's solution, 0.5% SDS, 50% formamide, and 32°C. The "moderately stringent conditions" are conditions involving, for example, 5 × SSC, 5 × Denhardt's solution, 0.5% SDS, 50% formamide, and 42°C. The "highly stringent conditions" are conditions involving, for example, 5 × SSC, 5 × Denhardt's solution, 0.5% SDS, 50% formamide, and 50°C. Under these conditions, it can be expected that DNA having higher homology is efficiently obtained in a higher temperature. However, possible factors that affect hybridization stringency may include a plurality of factors such as temperature, probe concentration, probe length, ionic strength, time, and salt concentration. Those skilled in the art can actually perform similar stringency by appropriately selecting these factors.

Example of the polynucleotide capable of hybridizing can include a polynucleotide having, for example, 70% or higher, 80% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity as compared with a control polynucleotide sequence, when the homology is calculated using default parameters in homology search software such as FASTA or BLAST. A larger numeric value of the homology is generally more preferred.

The identity or the homology between amino acid sequences or polynucleotide sequences can be determined using algorithm BLAST provided by Karlin and Altschul (Proc. Natl. Acad. Sci. USA 87 2264-2268, 1990; and Proc. Natl. Acad. Sci USA 90: 5873, 1993). Programs, called BLASTN or BLASTX, based on the BLAST algorithm have been developed (Altschul SF, et al: J Mol Biol 215: 403,1990). In the case of analyzing a nucleotide sequence using BLASTN, the parameters are set to, for example, score = 100 and word length = 12. In the case of analyzing an amino acid sequence using BLASTX, the parameters are set to, for example, score = 50 and word length = 3. In the case of using BLAST and Gapped BLAST programs, default parameters of each program are used.

### (5) Virus vector for facilitating blood coagulation function - (1)

The virus vector of the present invention can have a cleavage means alone for the target genome as the genome editing means in order to treat a hemorrhagic disease. The hemorrhagic disease may be ameliorated, for example, by exerting a distinctive alternative function or an auxiliary function instead of repairing a gene of a blood coagulation factor having a decline or deficiency in function in the hemorrhagic disease by the genome editing means described above. More specifically, when negative control in blood coagulation is stronger than positive control due to, for example, a reduced function of a blood coagulation factor resulting from a site-directed mutagenesis or deletion, the present invention provides a means in which the positive control is finally exerted by inhibiting or reducing the negative control of blood coagulation.

Examples of the anticoagulation factor (or blood coagulation control factor) known in the art include antithrombin, protein C, protein S, and tissue factor pathway inhibitor (TFPI). Accordingly, when a function of any of these factors is reduced in a patient having a reduced blood coagulation function, it can be expected that, the blood coagulation function is restored in the patient. Thus, as a means of inhibiting or reducing the negative control of blood coagulation, the virus vector of the present invention can adopt a means for reducing the function of the anticoagulation factor described above, for example, a means of non-homologous end repair capable of reducing a transcriptional or translational function for the anticoagulation factor in order to reduce an expression level thereof. For example, the virus vector of the present invention can specifically disrupt a genomic gene encoding antithrombin, protein C, protein S, or a combination thereof and reduce its function. More specifically, the virus vector of the present invention can comprise a means for editing a genomic gene of antithrombin and inhibiting or reducing its function, but is not limited thereto.

The antithrombin used in the present invention is a protein having a molecular weight of approximately 55 kDa and has, as a main anticoagulation function, a function of negatively controlling blood coagulation by inhibiting the step of converting a pro form of a factor to an active form through cleavage by thrombin, and further functionally inhibiting activated factor IX, X or VII. In the present invention, a protein that functions similarly to thrombin and negatively controls blood coagulation may instead be used.

The vector of the present invention is capable of providing a blood coagulation time equivalent to that of a healthy individual as a result of functionally inhibiting a protein negatively controlling blood coagulation. In the present specification, the blood coagulation time equivalent to that of a healthy individual means that an exhibited coagulation time is, for example, approximately 6 times, approximately 5 times, approximately 4 times, approximately 3 times, approximately 2.5 times, approximately 2 times, approximately 1.5 times, or approximately 1 or less time of the blood coagulation time of a healthy individual. The restoration of the blood coagulation function preferably means that the ability to form fibrin is approximately 10% or more, approximately 20% or more, approximately 30% or more, approximately 40% or more, approximately 50% or more, approximately 60% or more, approximately 70% or more, approximately 75% or more, approximately 80% or more, approximately 85% or more, approximately 90% or more, or approximately 95% or more as compared with a normal control.

### (6) Virus vector for repairing target gene - (2)

The virus vector(s) of the present invention can comprise a repair gene (repair means) in order to treat a hemorrhagic disease, preferably, in order to repair a blood coagulation factor having a decline or deficiency in function, together with the genomic cleavage means as the genome editing means. The virus vector(s) of the present invention may have both the genome editing means and the repair means in one virus vector, or alternatively, a vector (a1) comprising the genome editing means and a virus vector (a2) comprising the repair means may be separately contained in a plurality of vectors. Accordingly, the rAAV for genomic editing of the present invention may comprise one or two or more rAAV vectors. When the virus vector of the present invention comprises a plurality (two or more) of rAAV vectors, the plurality (two or more) of rAAV vectors may be concurrently administered or may be continuously administered. Preferably, the two or more rAAV vectors of the present invention are concurrently administered. The virus genome quantitative ratio (virus genome: vg) of these rAAV vectors may be in the range of 0.3 to 10, preferably 0.5 to 8, more preferably 1 to 5, in terms of repair means/genome editing means.

The virus vector (a2) comprising the repair means may comprise, for example, regions homologous to 5' and 3' sides of the cleavage target site in the genome on the 5' and 3' sides of the repair gene. These regions are homologous regions (arms) for homologous recombination at the target cleavage site. For the length of each of the arms, for example, approximately 100 bp, approximately 200 bp, approximately 300 bp or a larger length is available, while an upper limit of the length of up to approximately 3 kbp, for example, a length of approximately 3 kbp or smaller, approximately 2.5 kbp or smaller, approximately 2 kbp or smaller, approximately 1.5 kbp or smaller, or approximately 1 kbp or smaller is available, in view of the length of the gene capable of being inserted into AAV vectors.

This repair means may further comprise a polynucleotide encoding a normal blood coagulation factor or a part thereof between the arms. For designing the repair means of the virus vector of the present invention, it is necessary to pre-identify a gene or a gene portion of a blood coagulation factor involved in an abnormality in a patient, such as a decline or deficiency in function, . For such an identification method, a protein analysis technique, a gene diagnosis technique, or the others known in the art can be used. When a gene or a gene portion involved in a decline or deficiency in function is consequently identified, it is possible to design the repair means of the present invention in order to attain to repair or complement such a gene or gene portion. In one embodiment, the repair means of the present invention comprises a normal polynucleotide, or a part thereof, encoding a blood coagulation factor or the like involved in an abnormality such as a decline or deficiency in function, between the arms homologous to the 5' and 3' sides of the cleavage target site.

In one embodiment of the present invention, the virus vector of the present invention comprises a full-length gene encoding normal FIX (human IX NM_000133.3; mouse FIX NM_007979; NM_001305797) or a part thereof as the repair means. In this context, the polynucleotide encoding full-length normal FIX or a part thereof can complement or repair deficient or abnormal FIX, which contributes to the pathogenesis of hemophilia. As a result of the complement or the repair, normal FIX can be expressed in an amount effective for bringing about blood coagulation at the intended level to a patient. In a more specific embodiment of the present invention, the repair means comprises a polynucleotide comprising a normal exon, or a polynucleotide having a plurality of normal exons including the normal exon, in order to replace an exon containing a mutation site in a FIX coding region. Such a repair polynucleotide is delivered to a site of a target tissue (liver, etc.) by the vector of the present invention (a2) which comprises the repair means, and introduced (i.e., repaired) to the target site preferably via homologous recombination when gene editing is performed by the vector of the present invention (a1) which comprises the genome editing means and is separately delivered thereto. As a result of the introduction, the normal FIX is expressed at a level capable of providing the blood coagulation of interest. Specifically, as a result of introducing the repair gene of the present invention, preferably, improvement in blood coagulation function is achieved in a subject. The polynucleotide of the repair means may be subjected to codon optimization for differentiation from endogenous genes.

As used herein, the expression at a level capable of providing the normal function refers to, for example, expression at a level that provides blood coagulation in a time equivalent to the blood coagulation of a healthy individual. In the present specification, the blood coagulation time equivalent to that of a healthy individual means to show that, for example, the time until bleeding stops is shortened to approximately 6 times or more, approximately 5 times, approximately 4 times, approximately 3 times, approximately 2.5 times, approximately 2 times, approximately 1.5 times, or approximately 1 time less than the time until bleeding in a hemophilia individual stops, as described above. The restoration of the blood coagulation function means that the enzyme activity of a protein from the introduced repair gene (e.g., FIX) in an individual having said introduced repair gene is restored by 1% or more, 2% or more, 3% or more, 4% or more, 5% or more, 6% or more, 7% or more, 8% or more, 9% or more, approximately 10% or more, approximately 20% or more, approximately 30% or more, approximately 40% or more, approximately 50% or more, approximately 60% or more, approximately 70% or more, approximately 75% or more, approximately 80% or more, approximately 85% or more, approximately 90% or more, or approximately 95% or more as compared with the activity in a control (e.g., a healthy person). Alternatively, the restoration of the blood coagulation function means that, for example, the ability to form fibrin in an individual having the introduced repair gene is restored by approximately 10% or more, approximately 20% or more, approximately 30% or more, approximately 40% or more, approximately 50% or more, approximately 60% or more, approximately 70% or more, approximately 75% or more, approximately 80% or more, approximately 85% or more, approximately 90% or more, or approximately 95% or more as compared with a normal control.

The FIX used in the present invention refers to a polynucleotide encoding the same amino acid sequence as that of normal FIX, or a polynucleotide encoding an amino acid sequence that provides a specific activity equivalent thereto. In this context, the equivalent specific activity means, for example, approximately 40%, approximately 50%, approximately 60%, approximately 70%, approximately 75%, approximately 80%, approximately 85%, approximately 90%, or approximately 95% or more.

Examples of such an amino acid sequence include an amino acid sequence having deletion, substitution, insertion and/or addition of, for example, 1 to 50, 1 to 40, 1 to 39, 1 to 38, 1 to 37, 1 to 36, 1 to 35, 1 to 34, 1 to 33, 1 to 32, 1 to 31, 1 to 30, 1 to 29, 1 to 28, 1 to 27, 1 to 26, 1 to 25, 1 to 24, 1 to 23, 1 to 22, 1 to 21, 1 to 20, 1 to 19,1 to 18,1 to 17, 1 to 16,1 to 15, 1 to 14, 1 to 13, 1 to 12,1 to 11, 1 to 10, 1 to 9 (1 to several), 1 to 8, 1 to 7, 1 to 6, 1 to 5, 1 to 4, 1 to 3, 1 or 2, or 1 amino acid residues in the amino acid sequence of the normal FIX, and exhibits specific activity equivalent to the activity of FIX under physiological conditions.

Examples of the FIX used in the present invention include FIX that has an amino acid sequence having approximately 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity to the amino acid sequence of the normal FIX or an amino acid sequence having identity to the amino acid sequence of the normal FIX, and exhibits specific activity equivalent to the activity of FIX under physiological conditions.

Alternatively, a more upstream untranslated region may be selected as a cleavage site in the design of the genomic cleavage site. Several bases to several hundreds of bases may be deleted at the cleavage site during the cleavage or repair of the genome. Therefore, preferably, the cleavage site is designed so as to be disposed in an untranslated region or an intron region.

A method for designing a gene for use in conventional knock-out or knock-in can be used in the design of the gene for use in the repair means.

The sequences on both sides of the polynucleotide of the repair means used in the present invention, for example, the polynucleotide encoding full-length normal FIX or a part thereof, preferably have regions (arms) homologous to both sides of the target genomic cleavage site. The length of this homologous region can be appropriately selected as a length in the range of several tens of bases to several hundreds of bases. Preferably, the lower limit size can be appropriately selected from 100 or more bases, 150 or more bases, 200 or more bases, 250 or more bases, and 300 or more bases. Examples of the upper limit size include, but are not limited to, 2000 or less bases, 1500 or less bases, 1000 or less bases, 900 or less bases, 800 or less bases, 700 or less bases, 600 or less bases, 500 or less bases, 400 or less bases, 350 or less bases, and 300 or less bases.

For the repair, in order to reduce protein expression from the original endogenous genome, the repair gene may be differentiated between the repair gene and the original genome, for example, by codon substitution or codon optimization of the nucleotide sequence on the original genome resulting in a difference from the original endogenous genome, and may further comprise a means of inhibiting specific expression of the original genome. Examples of such inhibiting means include, but are not limited to, polynucleotides for changing (e.g., disrupting or reducing) the function of a target endogenous gene, and polynucleotides for changing (e.g., reducing) the expression level of the endogenous protein, such as antisense molecules, ribozyme, interfering RNA (iRNA), and microRNA (miRNA). For example, methods for preparing and using double-stranded RNA (dsRNA, siRNA, shRNA or miRNA) have been known in the art from many literatures (see JP-A-2002-516062; U.S. Patent Publication No. 2002/086356A; Nature Genetics, 24(2), 180-183, 2000 Feb., etc.).

### (7) Measurement and restoration of blood coagulation function

In the present invention, the blood coagulation function can be measured by confirmation using a method known in the art. For example, coagulation factor activity can be usually measured by a method referred to as one-stage coagulation assay (Eur J Haematol. 2015 Feb; 94 Suppl 77:3 8-44). Alternatively, a method using procedures described in "Material and procedure of experiment" "(9) Bleeding time" in Examples in the present specification can be used.

As used herein, the restoration of the blood coagulation function preferably means that the bleeding time of hemophilia is ameliorated, i.e., a blood coagulation time equivalent to that of a healthy individual, as described about FIX above, is exhibited, as a result of treatment.

### 2.3. Preparation of rAAV vector

A method for preparing the rAAV vector of the present invention can adopt a common method and can comprise, for example, the step of transfecting cultured cells with (A) a first polynucleotide encoding a capsid protein (which is generally referred to as an AAV helper plasmid) and (B) a second polynucleotide to be packaged into the rAAV vector of the present invention (which comprises a therapeutic gene of interest). The preparation method according to the present invention can further comprise the step of transfecting the cultured cells with (C) a plasmid called adenovirus (AdV) helper plasmid, encoding an adenovirus-derived factor, or the step of infecting the cultured cells with an adenovirus. Moreover, the preparation method can further comprise the steps of: culturing the cultured cells thus transfected; and collecting a rAAV vector from the culture supernatant. Such a method has already been known in the art and is also used in Examples in the present specification.

In the first polynucleotide (A), preferably, the nucleotide encoding the capsid protein of the present invention is operably linked to a promoter sequence which is known in the art and operable in cultured cells. For example, cytomegalovirus (CMV) promoter, EF-1α promoter, or SV40 promoter can be appropriately used as such a promoter sequence. The first polynucleotide (A) may appropriately further comprise an enhancer sequence, a Kozak sequence, a poly A addition signal sequence, and the like that are known in the art. However, a genome size that can be packaged in rAAV needs to be taken into consideration.

The second polynucleotide (B) comprises the genome editing means and/or the repair gene at a position operable with the hepatocyte-specific promoter described below. The second polynucleotide (B) may appropriately further comprise an enhancer sequence, a Kozak sequence, a poly A addition signal sequence, and the like known in the art. The first polynucleotide may further comprise a cloning site cleavable with various restriction enzymes known in the art, downstream of the hepatocyte-specific promoter sequence. A multicloning site comprising a plurality of restriction enzyme recognition sites is more preferred. Those skilled in the art can insert the genome editing means and/or the repair gene of interest to downstream of the hepatocyte-specific promoter according to genetically engineering procedures known in the art. For such genetically engineering procedures, see, for example, Molecular Cloning 3rd Edition, J. Sambrook et al., Cold Spring Harbor Lab. Press. 2001.

In the preparation of the rAAV vector of the present invention, the first polynucleotide (A) and the second polynucleotide (B) can be introduced at the same time into the cultured cells using a helper virus plasmid (e.g., from adenovirus, herpes virus or vaccinia). Preferably, the preparation method of the present invention further comprises the step of introducing an adenovirus (AdV) helper plasmid. In the present invention, preferably, the AdV helper is derived from a virus targeting the same species as the cultured cells. For example, in the case of using human cultured cells HEK293, a human AdV-derived helper virus vector can be used. A commercially available product (e.g., AAV Helper-Free System from Agilent Technologies, Inc. (catalog No. 240071)) can be used as such an AdV helper vector.

In the preparation of the rAAV vector of the present invention, various methods known in the art such as calcium phosphate method, lipofection, and electroporation can be used as a method for transfecting the cultured cells with one or more of the plasmids described above. Such a method is described in, for example, Molecular Cloning 3rd Ed., and Current Protocols in Molecular Biology, John Wiley & Sons 1987-1997.

### 3. Pharmaceutical composition of present invention

In further another embodiment of the present invention, a pharmaceutical composition including an rAAV vector (rAAV virion) of the present invention is provided. Provided is a pharmaceutical composition including the rAAV vector of the present invention (hereinafter, referred to as pharmaceutical composition of the present invention), by which a therapeutic gene can be introduced into hepatocytes of a subject with high efficiency, and the target disease can be treated via expression of the introduced therapeutic gene. Such an rAAV vector containing a therapeutic gene is included in the pharmaceutical composition of the present invention. Examples of the therapeutic gene include, but not limited to, the above-described genome editing means and genome repairing means.

In an embodiment, the rAAV vector of the present invention preferably includes a promoter sequence specific to liver cells and a gene operably liked to the promoter sequence. The rAAV vector of the present invention can include a gene useful for treatment and the like, and can introduce such a gene into liver cells, preferably into hepatocytes.

The pharmaceutical composition of the present invention can be used through, for example, an oral, parenteral (intravenous), muscular, oral mucosa, rectal, vaginal, transdermal, nasal, or inhalation route, and parenteral administration is preferred. Intravenous administration is further preferred. The active ingredient(s) of the pharmaceutical composition of the present invention may be contained alone or formulated in combination, or may be provided in a pharmaceutical formulation in which a pharmaceutically acceptable carrier or an additive for pharmaceutical preparation is combined. In such a case, the content of the active ingredient of the present invention in the pharmaceutical preparation can be, for example, 0.1 to 99.9 wt%.

The active ingredient(s) of the pharmaceutical composition of the present invention may be contained alone or formulated in combination, or may be provided in a pharmaceutical formulation in which a pharmaceutically acceptable carrier or an additive for pharmaceutical preparation is contained. In such a case, the content of the active ingredient(s) of the present invention in the pharmaceutical preparation can be, for example, 0.1 to 99.9 wt%. As the pharmaceutically acceptable carrier or additive, for example, an excipient, a disintegrant, a disintegration assistant, a binder, a lubricant, a coating agent, a dye, a diluent, a solubilizer, a solubilizing assistant, an isotonic agent, a pH adjuster, or a stabilizer can be used.

Examples of the pharmaceutical preparation suitable for oral administration include powders, tablets, capsules, fine granules, granules, liquids, and syrups. In oral administration, various excipients, such as microcrystalline cellulose, sodium citrate, calcium carbonate, dipotassium phosphate, and glycine, can be used together with a starch, preferably a corn, potato, or tapioca starch, various disintegrants, such as alginic acid and certain silicic acid double salts, and a granule-forming binder, such as polyvinyl pyrrolidone, sucrose, gelatin, or gum Arabic. Lubricants, such as magnesium stearate, sodium lauryl sulfate, and talc, are very effective for tablet formation in many cases. Solid compositions of the same type can also be used by be filled in gelatin capsules. Examples of suitable related material include high molecular weight polyethylene glycol, in addition to lactose or sucrose. In order to prepare an aqueous suspension and/or an elixir for oral administration, an active ingredient is used in combination with a variety of sweeteners or flavors and coloring agents or dyes and, as needed, an emulsifier and/or a suspending agent, and can be used together with a diluent, such as water, ethanol, propylene glycol, glycerol, or a combination thereof.

Examples of the pharmaceutical preparation suitable for parenteral administration include injections and suppositories. In parenteral administration, a solution prepared by dissolving the active ingredient of the present invention in either sesame oil or peanut oil or dissolving the active ingredient in a propylene glycol aqueous solution can be used. The aqueous solution is appropriately buffered as needed (preferably a pH of 8 or more), and the liquid diluent needs to be first made isotonic. As such a liquid diluent, for example, physiological saline can be used. The prepared aqueous solutions are suitable for intravenous injection, while the oil solutions are suitable for intraarticular, intramuscular, and subcutaneous injection. Production of these all solutions under aseptic conditions can be easily achieved by standard pharmaceutical techniques well known to those skilled in the art. Furthermore, it is also possible to locally administer the active ingredient of the present invention to, for example, skin. In such a case, local administration in a cream, jelly, paste, or ointment form is desirable in accordance with a standard pharmaceutical practice.

The dose of the pharmaceutical composition of the present invention is not particularly limited, and an appropriate dose can be selected depending on various conditions, such as the type of disease, the age and symptoms of the patient, the administration route, the purpose of treatment, and presence or absence of other concomitant drug. The dose of the pharmaceutical composition of the present invention is, for example, 1 to 5000 mg per day for an adult (e.g., weight: 60 kg), preferably 10 to 1000 mg, but is not limited to these doses. Such a daily dose may be administered in two to four divided doses. When the administration unit is vg (vector genome), the dose per kg of body weight can be selected, for example, within a range of 10⁹ to 10¹⁴ vg, preferably 10¹⁰ to 10¹³ vg, further preferably 10¹⁰ to 10¹² vg, but not limited thereto.

The pharmaceutical composition of the present invention can be used in combination with a known drug not including a genome editing means for treating blood coagulation-related diseases caused by mutations on the genome of the liver, including hemophilia, factor VII deficiency, factor XI deficiency, antithrombin deficiency, protein S abnormality/deficiency, and protein C abnormality, but not limited thereto. Examples of the drug include, but not limited to, plasma-derived FVIII, plasma-derived FVII/VWF, desmopressin acetate, plasma-derived FIX, recombinant FIX, and recombinant FVIIa.

### 4. Administration of virus vector of present invention

The virus vector of the present invention is preferably administered to a subject by peripheral administration more safely and easily. In the present specification, the term "peripheral administration" refers to an administration route usually understood as peripheral administration by those skilled in the art, such as intravenous administration, intraarterial administration, intraperitoneal administration, intracardiac administration, and intramuscular administration.

The virus vector of the present invention administered to a subject infects liver cells and provides the genome editing means delivered by the virus with the infected cells and can consequently bring genome editing. The virus vector of the present invention preferably infects hepatocytes and can bring genome editing. In the virus vector of the present invention, the proportion of the genome edited cells in the hepatocytes of the liver of the administered subject is preferably 70% or more, 80% or more, 85% or more, 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, 99.9% or more, or 100%.

### 5. Kit for preparing rAAV vector of the present invention

Another embodiment of the present invention provides a kit for producing rAAV of the present invention. Such a kit can include, for example, (a) a first polynucleotide for expressing capsid protein VP1 or the like and (b) a second polynucleotide to be packaged in the rAAV vector. For example, the first polynucleotide comprises a polynucleotide encoding the amino acid of SEQ ID NO: . For example, the second polynucleotide may comprise the target therapeutic gene or not and can preferably contain various restriction enzyme cleavage sites for incorporating such a target therapeutic gene.

The kit for producing the rAAV virion of the present invention can further include any of the components (such as AdV helper) described in the present specification. The kit of the present invention can further include an instruction describing the protocol for producing the rAAV virion by the kit of the present invention.

### 6. Other terms herein

The meanings of terms used herein are as follows.

As used herein, the terms "virus vector", "viral virion", and "viral particle" are mutually exchangeable, unless otherwise stated.

As used herein, the term "polynucleotide" is exchangeable with "nucleic acid", "gene", or "nucleic acid molecule" and refers to a polymer of nucleotides. As used herein, the term "nucleotide sequence" is exchangeable with "nucleic acid sequence" or "base sequence" and refers to a sequence of deoxyribonucleotides (abbreviated as A, G, C, and T). For example, the "polynucleotide comprising the nucleotide sequence set forth in SEQ ID NO: 1 or a fragment thereof" refers to a polynucleotide comprising the sequence indicated with the deoxynucleotides A, G, C, and/or T in SEQ ID NO: 1 or a fragment thereof.

The "virus genome" and "polynucleotide" according to the present invention may each be present in a form of DNA (for example, cDNA or genomic DNA), but may optionally be in a form of RNA (for example, mRNA). The virus genomes and polynucleotides used herein may each be double or single stranded DNA. When they are single stranded DNA or RNA, they may be a coding strand (known as a sense strand) or a non-coding strand (known as an antisense strand).

Unless specifically stated, the description of genetic arrangement of a promoter, a gene of interest and a polyadenylation signal encoded by the rAAV genome herein where the rAAV genome is a sense strand refers to the sense strand itself, and the description of such genetic arrangement where the rAAV genome is an antisense strand refers to the complementary strand thereof. Moreover, when it is apparent in the context, "r" that represents recombinant may be omitted herein.

As used herein, the terms "protein" and "polypeptide" are mutually exchangeable and refers to a polymer of amino acids. The polypeptides as used herein means that the N-terminal (amino terminal) is on the left end and the C-terminal (carboxyl-terminal) is on the right end, according to the customary notation of peptide. The partial peptide of the polypeptide according to the present invention (which may be abbreviated herein as the partial peptide of the present invention) refers to a partial peptide of the aforementioned polypeptide according to the present invention preferably having the same properties as the aforementioned polypeptide according to the present invention.

As used herein, the term "plasmid" means various known genetic elements, for example, a plasmid, a phage, a transposon, a cosmid, or a chromosome. The plasmid can be replicated in a certain host and can transfer a genetic sequence between cells. As used herein, the plasmid includes various known nucleotides (DNA, RNA, PNA, and a mixture thereof). The plasmid may be single stranded or double stranded, but preferably double stranded. For example, as used herein, the term "rAAV vector plasmid" is intended to include a double strand formed of an rAAV vector genome and a complementary strand thereof, unless otherwise specified. The plasmid used in the present invention may be linear or circular.

As used herein, the term "packaging" refers to a phenomenon which comprises the steps of, for example, preparing single stranded virus genome, assembling coat proteins (capsid), and packaging the virus genome with capsids (encapsidation). When appropriate plasmid vectors (usually plasmids) are introduced into a cell line in which the packaging can be performed under appropriate conditions, recombinant viral particles (i.e., viral virions or virus vectors) are assembled and secreted into the culture medium.

As used herein, terms that are not described specifically are intended to refer to a range of meanings of the terms that those skilled in the art usually understand.

### [Examples]

Hereafter, the present invention will be further specifically described by the working examples, but the scope of the present invention is not limited to the following examples.

### 1. Experimental material and method

### (1) Experimental animal

All animal protocols were approved by the Institutional Animal Care and Concern Committee at Jichi Medical University, and animal care was in accordance with the committee's guidelines. C57BL/6J mice were purchased from Japan SLC, Inc. Coagulation factor IX (FIX)-deficient mice (B6.129P2-F9^{tm1Dws}) were obtained from The Jackson Laboratory (USA).

### (2) Design of guide RNA (sgRNA) sequence and DNA construct

sgRNA sequences were designed using online software provided by Thermo Fisher Scientific, Inc. (Waltham, MA, USA) or Benchling (https://benchling.com) (Curr. Issues Mol. Biol., 2016, 20: 1-12). The designed gRNAs are shown in the following table (SEQ ID NOs: 3 to 5 and 7 to 15). The DNAs encoding gRNAs (trans-activating RNA-crispr RNA chimera) under the control of the U6 promoter were synthesized by GenScript Inc. (USA). PCR fragments of the sgRNA sequences without the U6 promoter were inserted into plasmid vector pCR2.1 TOPO, and the sgRNAs were generated by *in vitro* transcription (CUGA7 *in vitro* Transcription Kit (Nippon Gene Co., Ltd., Catalog No. 304-14641/307-13531).

### [Table 1]

**Table 1. List of sgRNA sequences and PAM sequences**

| Target gene | Cas9 | sgRNA | Target site | Sequence | PAM sequence |
|---|---|---|---|---|---|
| *F9* | *Streptococcus pyogenes* | sgRNA-1 | Exon 8 | GTCTTCAACAAAGGGAGAC* | AGG |
| | | sgRNA-2 | Exon 8 | CAGTACCTTAGAGTTCCAC | TGG |
| | | sgRNA-3 | Exon 8 | GGTTTCCCGGTACGTCAAC | TGG |
| *F9* | *Staphylococcus aureus* | sgRNA-1 | Exon 8 | TCAACAAAGGGAGACAGGCTT | CCATTC |
| | | sgRNA-2 | Exon 8 | CAGTACCTTAGAGTTCCACTG | GTGGAT |
| | | sgRNA-3 | Exon 8 | TAAGGTTTCCCGGTACGTCAA | CTGGAT |
| *F9* | *Staphylococcus aureus* | sgRNA-1 | Intron 1 | TTGATCCCGAGGGTCTATACA | GTGAAT |
| | | sgRNA-2 | Intron 1 | CAGGAGACCAGCCGATTTTCT | GGGGAT |
| | | sgRNA-3 | Intron 1 | TCCCTCACCACTAAGACGTGC | TTGGAT |
| *Serpinc1 (antithrombin)* | *Staphylococcus aureus* | sgRNA-1 | Exon 8 | GAGGAAGGCAGTGAAGCAGCA | GCGAGT |
| | | sgRNA-2 | Exon 3 | CCTGGGAAGAAGGCCACCGAG | GAGGAT |
| | | sgRNA-3 | Exon 3 | ACTGCTTTTGCTATGACCAAG | CTGGGT |

| | | | | | |
|---|---|---|---|---|---|
| * Shared sequences of sgRNAs between SpCas 9 and SaCas 9 (underlined). | | | | | |

### (3) Generation of CRIPSR/Cas9-mediated gene-modified mice

Codon-optimized Streptococcus pyogenes Cas9 (SpCas9) mRNA (20 ng/µL; Thermo Fisher Scientific, Inc.) and an sgRNA (5 ng/µL) were injected into the cytoplasm of zygotes. The zygotes were cultured until the two-cell stage, and then transferred into pseudopregnant female mice.

### (4) Surveyor® nuclease assay

Genetic mutations were determined using a Surveyor® Mutation Detection Kit (Integrated DNA Technologies, Inc.). Briefly, PCR fragments were amplified using ExTaq DNA polymerase (Takara Bio Inc.). Equal amounts of test and reference PCR products were denatured and re-annealed using a thermal cycler, and then treated with Surveyor® nuclease. DNA fragments were analyzed by agarose gel electrophoresis. The oligonucleotide primer sequences used to detect mutations are described in the following table 2 (SEQ ID NOs: 17 to 33).

### [Table 2]

**Table 2. List of used primer pairs**

| Target DNA | | Sequence |
|---|---|---|
| Surveyor Assay | | |
| Exon 8 of mouse *F9* | F | 5'-AACTGGGCAAATGGGAGAG-3' |
| | R | 5'-TCAGGAGAGGAAGTCATGC-3' |
| Intron 1 of mouse *F9* | F | 5'-AACAGTGGCATTACTCCCCA-3 |
| | R | 5'-CCAAACTGGCCTGTGAGAAA-3 |
| Exon 8 of mouse *Serpinc1* | F | 5'-GTGGTAGTGATAGCTGGGAT-3' |
| | R | 5'-GGGAGATTGATTCGGGTTTG-3' |
| Exon 3 of mouse *Serpinc1* | F | 5'-CCTTGTCTGGGGTTTTCTGA-3' |
| | R | 5'-GATCACTGGGTGTCTTTCCA-3' |
| Real time qPCR | | |
| SV40 polyA | F | 5'-AGCAATAGCATCACAAATTTCACAA-3' |
| | R | 5'-CCAGACATGATAAGATACATTGATGAGTT-3' |
| | probe | 5'-AGCATTTTTTTCACTGCATTCTAGTTGTGGTTTGTC-3' |
| Deep sequencing | | |
| Exon 8 of mouse *F9* | F | 5'-TCGTCGGCAGCGTCAGATGTGTATAAGAGACAGTGATCAGTGAAGCCAACCAGACTGGG-3'* |
| | R | 5'-GTCTCGTGGGCTCGGAGATGTGTATAAGAGACAGCCTTCACACGAATCTTTGCCTCCTTC-3'* |
| Detection of HDR and NHEJ | | |
| | F | 5'-GGGATCTACACCAAAGTGAG-3' |
| | R | 5'-CCAAACTGGCCTGTGAGAAA-3 |
| Detection of coF9 mRNA | | |
| | F | 5'-ATGAAGCACCTGAACACCGT-3' |
| | R | 5'-CCAGTTCACGTATCTGCTCA-3' |

| | | |
|---|---|---|
| * Overhang adapter sequences appended to the primer pair sequence (underline). coF9, codon-optimized F9. | | |

### (5) Plasmid constructs and AAV vector production

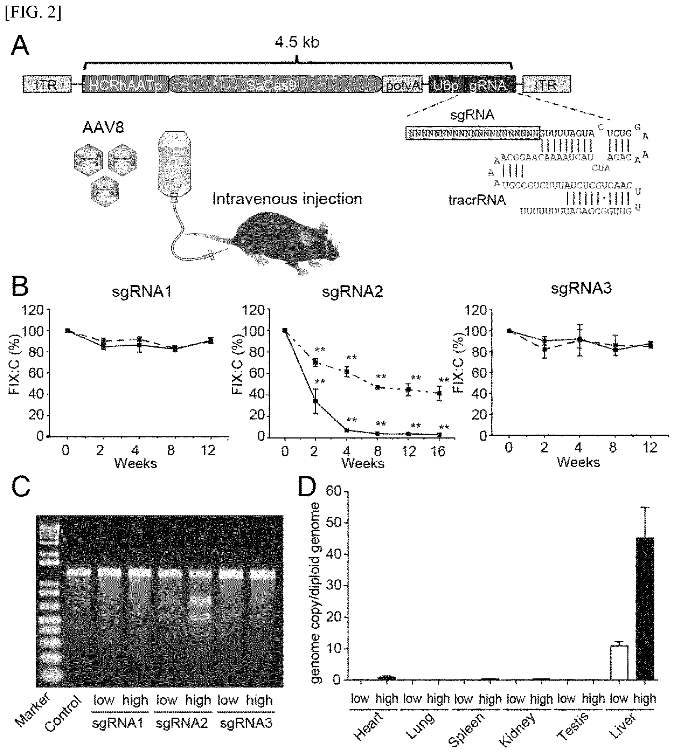

The cDNA of codon-optimized Staphylococcus aureus Cas9 (SaCas9) (Ran, et al., 2015) was provided from Dr. Nureki of The University of Tokyo. A chimeric promoter (HCRhAAT: an enhancer element of the hepatic control region of ApoE/C1 gene and the human anti-trypsin promoter) (Mimuro, et al., 2013), SaCas9 cDNA, the SV40 polyadenylation signal, and an sgRNA sequence driven by the U6 promoter were introduced between the inverted terminal repeats of the pAAV2 plasmid (FIG. 2A). The genes were packaged by triple plasmid transfection of human embryonic kidney 293 cells (HEK 293) to produce a recombinant AAV8 vector (helper free system) by the previously described method (Mimuro, et al., 2013). Titration of each of recombinant AAV vectors was carried out by quantitative PCR.

### (6) Targeted deep sequencing

Genomic DNA fragments that include the nuclease target sites were amplified using a KAPA HiFi™ HotStart PCR kit (KAPA Biosystems, Inc., Wilmington, MA, USA). The PCR products were purified using AMPure® XP beads (Beckman Coulter, Inc., Brea, CA, USA). A library was prepared to amplify the target region and to add Illumina sequencing adapters and dual-index barcodes to the amplicon target. PCR amplicons were subjected to 300 pair-end read sequencing using Illumina MiSeq (100,000 reads). Sixty sequences near the target sequence were extracted, and the frequency of each sequence was calculated.

### (7) Measurement of coagulation factor activity and thrombin generation assay

Plasma FIX:C (blood coagulation factor IX activity) was measured with a one-stage clotting-time assay by the automated coagulation analyzer (Sysmex CA-510 analyzer: Sysmex Corporation) using FIX-deficient plasma (Sysmex Corporation). AT:C was measured using Testzym® S ATIII (Sekisui Medical Co., Ltd.). Thrombin generation was assessed as previously reported (Matsumoto, et al., 2009). Briefly, 80 µL of mouse plasma was incubated with 20 µL of a trigger reagent (the mixture of 5 pM recombinant human tissue factor and 40 µM phospholipid) for 10 minutes at 37°C. The assay was started by the addition of 20 µL of 100 mM CaCl₂ and 5 mM of thrombin substrate Z-Gly-Gly-Arg-AMC (Wako Pure Chemical Industries). A fluorescent signal (excitation: 390 nm and emission: 460 nm) was monitored at 10 second-intervals using a Spark 10M multimode microplate reader (Tecan Group Ltd., Männedorf, Switzerland).

### (8) Histological analysis

Mice anesthetized with isoflurane were perfused with 50 mL of phosphate buffered saline, and then tissues were fixed with 10% formalin. Paraffin-embedded tissue samples were sectioned and processed for hematoxylin-eosin staining or immunostaining. The SaCas9 used in this study was conjugated with hemagglutinin (HA). The sections were pretreated with 5% donkey serum, and then treated with anti-HA polyclonal antibody (Medical & Biological Laboratories Co., Ltd.). Immunoreactivity was detected with Simple Stain Mouse MAX-PO (Nichirei Biosciences Inc.) and DAB (Agilent Technologies, Inc.), followed by Myer hematoxylin. The tissue sections were observed with an all-in-one microscope at 400× magnification.

### (9) Bleeding time

The distal tail tip (5 mm) of an anesthetized mouse was clipped, and the tail was immediately immersed in 50 mL of phosphate buffered saline at 37°C. The tail bleeding times were defined as the time required for hemostasis. When bleeding time went beyond 10 minutes, the experiment was terminated by electrocauterization to prevent the animal death.

### (10) Intravital microscopy

Intravital microscopy was performed to analyze fibrin formation *in vivo.* Briefly, Rhodamine B isothiocyanate-dextran (5 mg/body, Sigma Aldrich Co. LLC), Hoechst 33342 (3 mg/body, Thermo Fisher Scientific, Inc.), and Alexa 488-conjugated fibrinogen (300 µg/body, Thermo Fisher Scientific, Inc.) were injected into anesthetized mice. Sequential images of testicular vein (diameter: at least 80 µm) were obtained using a resonance scanning confocal microscope (Nikon A1RNP, Nikon Corporation) after local endothelial disruption induced by laser irradiation (wavelength: 700 nm). The signal intensity of fibrin formation (shown by signals of Alexa 488-conjugated fibrinogen) was quantified using NIS-Elements AR 3.2 (Nikon Corporation).

### (11) Statistical analysis

Unless otherwise stated in the present specification, numerical values express mean ± standard deviation (SEM). Statistical analysis was performed by Student's t-test or one-way repeated-measures ANOVA with *post hoc* Bonferroni test.

### 2. Experimental results

### (1) Production of FIX deficiency mice

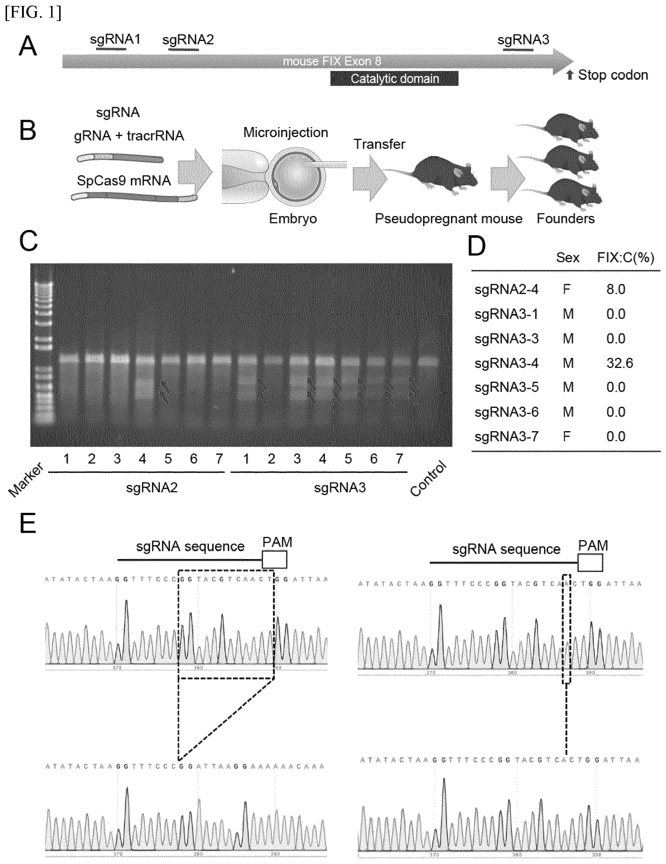

First, mice with hemophilia B were generated using CRISPR/Cas9. The gene of exon 8 of F9 were disrupted, where the majority of mutations in hemophilia B patients are located (Li, et al., 2013). Streptococcus pyogenes Cas9 (SpCas9) mRNA and sgRNAs specific to exon 8 of mouse F9 were injected into fertilized embryos (FIGs. 1A and 1B). The sequences of the used sgRNAs (including tracrRNA portion, crRNA portion, and protospacer adjacent motif (PAM) sequences) are shown in SEQ ID NOs: 3 to 5 and 7 to 9.

Newborn mice were assessed for F9 mutation by Surveyor® nuclease assay. As shown in FIG. 1, mutant mice were by two types of sgRNA produced. Cleavage of the target DNA was most efficiently induced by sgRNA3 (6/7: about 86%) (FIG. 1C). All mice carrying a mutation showed reduced plasma FIX:C (FIG. 1D). Plasma FIX:C was markedly reduced, even in female mice having two X chromosome alleles that have been cleaved. Some of these mice were bred to obtain offspring mice. DNA sequencing of two strains of male F2 mice not showing FIX activity (FIX:C) revealed deletion of 1 to 12 bases in the sgRNA sequence (FIG. IE).

### (2) Disruption of F9 in adult mice using an AAV vector

Next, mice with hemophilia were generated by disrupting F9 in adult mice via administering a virus vector. FIX is a vitamin K-dependent coagulation factor produced in the liver. The AAV8 vector has high efficiency for gene transfer to hepatocytes and is used in gene therapy for human hemophilia B (Gao, et al., 2002; Nathwani, et al., 2014; Nathwani, et al., 2011).

The AAV vectors are reported to usually accept 5 kb at most including promoter region (Wu, et al., 2010), it is generally difficult to incorporate the cDNA of SpCas9, which has a length of approximately 4.5 kb, into the AAV vectors. Therefore, the present inventors used Staphylococcus aureus Cas9 (SaCas9) having a smaller size, 3.2 kb (Ran, et al., 2015). The present inventors prepared a recombinant AAV8 vector that expresses SaCas9 in a liver-specific manner under control of a chimeric promoter (HCRhAAT, including an enhancer element of the hepatic control region of the Apo E/C1 gene and the human anti-trypsin promoter) and simultaneously expresses sgRNA under U6 promoter control (FIG. 2A). Because the PAM sequence of SaCas9 is different from that of SpCas9 (Ran, et al., 2015), the sgRNA sequence adjusted in-house was used.

The present inventors prepared AAV8 vectors expressing three types of sgRNA sequences, and intravenously injected them separately into 7-week-old C57BL/6J mice (FIG. 2A). The AAV8 vector expressing sgRNA2 led to reduction of plasma FIX:C in a vector dose-dependent manner (FIG. 2B). The levels of FIX: C decreased to 2% to 5% after the administration of high-dose vector (1×10¹²/body). The reduction of FIX:C lasted for at least 4 months (FIG. 2B). Other two sgRNA sequences did not reduce the plasma levels of FIX:C. The mutation in DNA derived from liver tissue was verified by Surveyor® nuclease assay (FIG. 2C).

### (3) Organ tropism by AAV8-mediate SaCas9 expression

The presence of the intravenously administered AAV8 vector was examined in various organs. The amounts of DNA derived from the AAV in organs were evaluated by real-time PCR. The AAV genome was mainly detected in the liver (FIG. 2D). The AAV DNA was also observed in the heart at a low level. However, no cleavage of the target genomic DNA was observed in any organ other the liver. The genomic DNA in the liver, which received a high-dose of the AAV8 vector expressing sgRNA2, was subjected to next-generation sequencing to calculate the frequency of mutations in the sequence. The most frequent mutations exibited deletions or replacement of several nucleotides near the PAM sequence (Table 3). The normal sequence was detected in 33.4% of the PCR amplicons (Table 3). As compared with the FIX:C results (2% to 5%), the frequency of the wild-type allele was relatively high. This result is probably because the liver contains endothelial cells and macrophages, to which gene transfer with AAV8 is difficult, in addition to hepatocytes. Immunohistochemical staining of SaCas9 expression in the liver demonstrated that SaCas9 was expressed in nearly all hepatocytes in the high-dose vector administration group. SaCas9 was mainly observed in the nuclei of hepatocytes, and was also observed in the nuclei of endothelial cells. No histological abnormality was detected in hematoxylin staining and eosin staining.

### [Table 3]

**Table 3. Frequency of F9 genomic sequences in liver without or with the administration of AAV vector encoding SaCas9 and sgRNA for F9 (Exon 8).**

| Genomic DNA sequence | Control (%) | sgRNA2 (%) |
|---|---|---|
| AAGGGAGACAGGCTTCCATTCTTCAGTACCTTAGAGTTCCACTG**GTGGAT**AGAGCCACAT | 88.7 | 33.4 |
| AAGGGAGACAGGCTTCCATTCTTCAGTACCTTAGAGTTCCA--G**GTGGA**TAGAGCCACAT | 0 | 19.8 |
| AAGGGAGACAGGCTTCCATTCCATTCTTCAGTACCTTAGAGTTCCA-TG**GTGGA**TAGAGCCACAT | 0 | 6.38 |
| AAGGGAGACAGGCTTCCATTCTTCAGTACCTTAGAGTTCCAACTG**GTGGA**TAGAGCCACAT | 0 | 5.06 |
| AAGGGAGACAGGCTTCCATTCTTCAGTACCTTAGAGTTCCA---**GTGGA**TAGAGCCACAT | 0 | 3.33 |
| AAGGGAGACAGGCTTCCATTCTTCAGTACCTTAGAGTTC--CTG**GTGGA**TAGAGCCACAT | 0 | 2.29 |
| AAGGGAGACAGGCTTCCATTCTTCAGTACCTTAGAGTT---CTG**GTGGA**TAGAGCCACAT | 0 | 1.52 |
| AAGGAGAGACAGGCTTCCATTCTTCAGTACCTTAGAGTTCCATCTG**GTGGA**TAGAGCCACAT | 0 | 1.36 |
| AAGGGAGACAGGCTTCCATTCTTCAGTACCTTAGAGTTCCA----**TGGAT**AGAGCCACAT | 0 | 1.13 |
| AAGGGAGACAGGCTTCCATTCTTCAGTACCTTAGAGTTCCA------**GAT**AGAGCCACAT | 0 | 1.09 |

| | | |
|---|---|---|
| * Table shows sequences a frequency of more than 1%. Underline and bold mean sgRNA and PAM sequence, respectively. | | |

### (4) Marginal increase in plasma FIX level by HDR

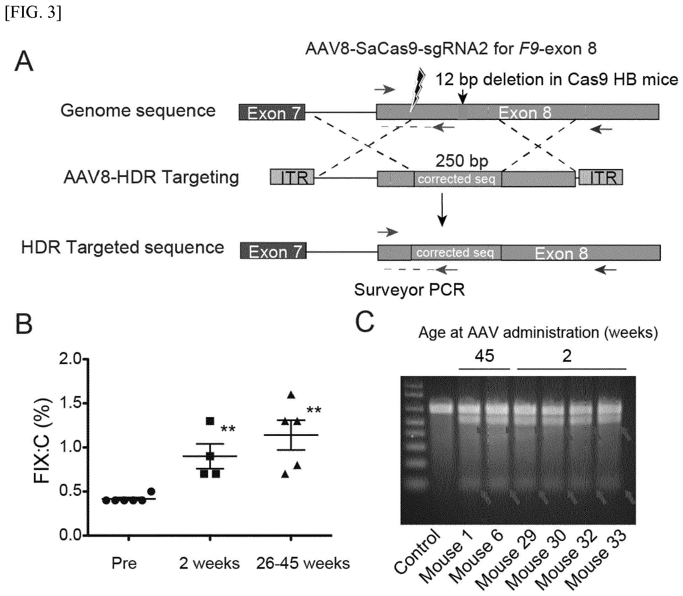

Following confirmation of the highly efficient cleavage of F9 in hepatocytes by CRISPR/Cas9, it was examined whether the delivery of a donor sequence for repairing the mutation simultaneously with the cleavage would lead to an increase in FIX:C through gene repair by HDR in hemophilia B mice generated by CRISPR/Cas9 (FIG. 1). The genome in these mice has an intact site recognized by sgRNA2 that efficiently produce DSBs using the AAV8 vector (FIG. 3A). A donor sequence with homology arms of 350 to 550 bp on both the 5' and 3' sides of the sgRNA2 sequence was incorporated into the AAV8 vector (FIG. 3A). Silent mutations were incorporated into the sgRNA2 of the donor sequence so that the homologously-recombined F9 would not be re-cleaved by SaCas9. The AAV8 vectors containing the donor sequence and expressing sgRNA2 and SaCas9 were simultaneously injected into juvenile or adult mice (2 or 26 to 46-week old). The FIX:C detected after the vector injection was marginally increased (FIG. 3B). It was demonstrated that, at the sites undergoing gene cleavage by SaCas9, gene repair occurred mainly by NHEJ, because mutations were detected in liver genomic DNA after the administration of the AAV vector by the Surveyor® nuclease assay.

### (5) Insertion of exon 2 to 8 cDNA into intron 1 of F9 for increasing FIX

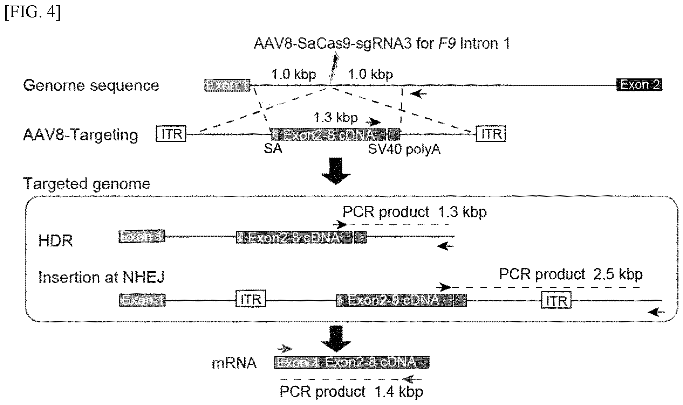

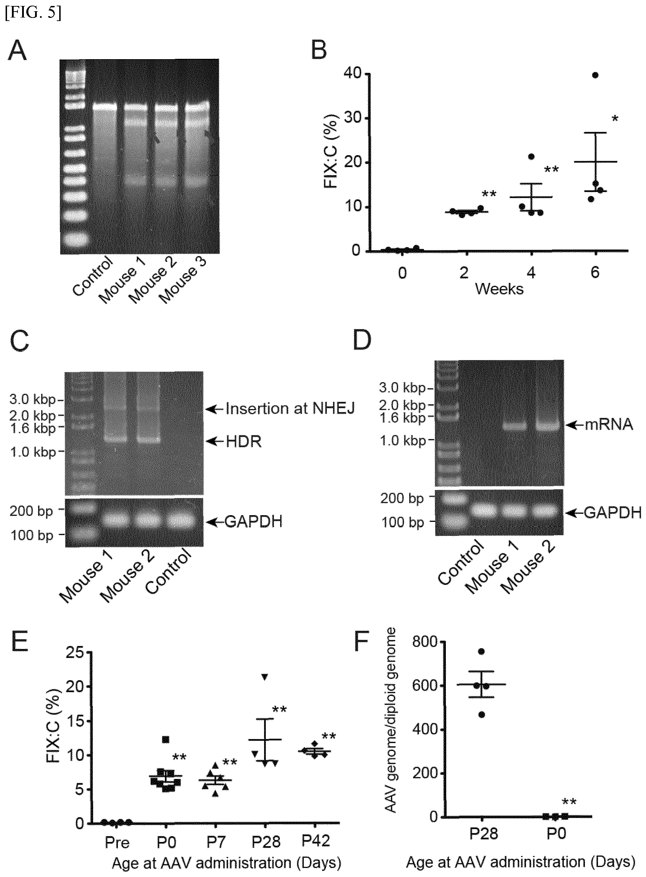

Previous studies using Zinc finger nucleases (ZFNs) suggested that a DNA fragment could be inserted into a double-strand break (DSB) at a safe locus by HDR and NHEJ (artificial insertion of human F9 at Rosa26 or A1b) (Anguela, et al., 2013; Sharma, et al., 2015). In order to insert a cDNA fragment into the endogenous F9 locus (FIG. 4) by a CRISPR/Cas9 system, the present inventors designed three types of sgRNA in intron 1 of F9 and confirmed the sgRNA3 for intron 1 effectively induced a DSB. Accordingly, sgRNA3 for intron 1 was inserted into the AAV8 vector system expressing SaCas9 (FIG. 4: AAV8-SaCas9-sgRNA3 for F9 intron), and it was found that sgRNA3 effectively induced a DSB in liver DNA after the administration of the AAV8 vector (FIG. 5A). Subsequently, a chimeric DNA sequence (human F9 splice acceptor site and codon-optimized mouse F9 cDNA (exons 2 to 8)) containing homology arms of 1 kb on both sides was designed, and was inserted into an AAV8 vector (FIG. 4: AAV8-Targeting). In order to insert the DNA sequence into the DSB site generated by SaCas9, AAV8-SaCas9-sgRNA3 for F9 intron (1×10¹² gc/body) and AAV8-Targeting (3×10¹² gc/body) were simultaneously administered to 8-week old adult hemophilia B mice produced by CRISPR/Cas9. As shown in FIG. 5B, FIX:C gradually increased to a level of 11.7% to 39.6%. In order to examine the mode of genome editing, the DNA sequence between the inserted cDNA and the outside of the 3' side arm (the arrow portion in FIG. 4) was amplified, and it was found that the F9 cDNA sequence was inserted into the DSB not only by HDR but also by NHEJ (FIG. 5C). Expression of FIX mRNA consisting of exon 1 and codon-optimized exons 2 to 8 was also observed after the vector administration (FIG. 5D). The administration of single AAV8-Targeting resulted in a negligible increase in FIX:C (data not shown). This result indicates that a DSB by SaCas9 is required for the treatment effects. Furthermore, the optimal period of the AAV-mediated genome editing was identified by comparing the timing of systemic administration of the AAV vector. As a result, as shown in FIG. 4E, similar treatment effects were observed at postnatal day 0 (P0), day 8 (P8), day 28 (P28), and day 42 (P42). The number of copies of the AAV genome in the liver at 8 to 16 weeks after the vector injection was significantly lower than that in mice treated at birth (P28: 604.5 ± 58.73, P0: 1.177 ± 0.3437, FIG. 5F). These results suggest that the treatment effect of genome editing is maintained even if the AAV genome is diluted by cell division during proliferation of hepatocytes.

### (6) Phenotypic correction of hemophilia by disruption of Serpinc1

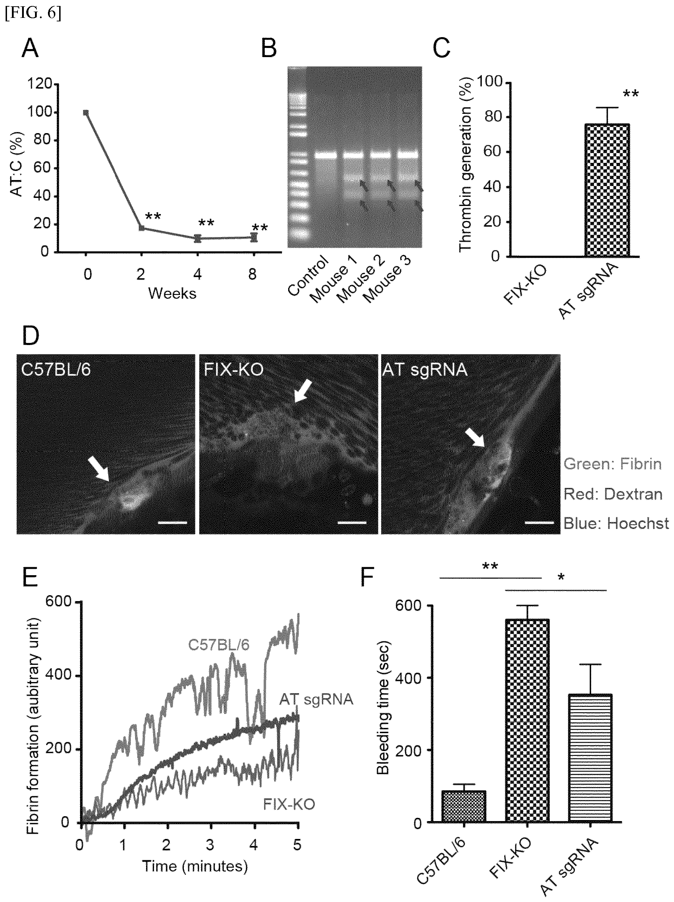

It was examined whether bleeding of hemophilia mice can be cured through disruption of the AT gene (Serpinc1) by CRISPR/Cas9 for treating the bleeding tendency of hemophilia using a single AAV vector system. Three types of sgRNA for Serpinc1 were designed, and a sequence that efficiently cleaves Serpinc1 (sgRNA1 for mAT) was selected (data not shown). AAV8 carrying the sgRNA and the SaCas9 was intravenously injected into FIX-deficient mice (hemophilia B mice). Plasma antithrombin (AT) activity (AT:C) clearly decreased after the injection of the vector (FIG. 6A), and DSBs in liver DNA after the administration of the vector were observed (FIG. 6B). This reduction of AT improved plasma thrombin generation *ex vivo,* fibrin formation after endothelial disruption *in vivo,* and bleeding time after tail clipping (FIGs. 6C to 6F). This demonstrates that the inhibition of an anticoagulant factor, such as AT, using CRISPR/Cas9 can be an alternative strategy for treating hemophilia.

### 3. Detailed description of drawings

FIGs. 1A to 1E show generation of hemophilia B mice by injection of sgRNA and SpCas9 mRNA into zygotes.

FIG. 1A shows a schematic diagram of sgRNA targeting exon 8 of F9.

FIG. 1B shows a method to generate CRSPR/Cas9-mediated hemophilia B mice, where sgRNA and SpCas9 mRNA were injected into zygotes and transferred into pseudopregnant female mice.

FIG. 1C shows that Cas9-mediated cleavage of F9 in founder mice was detected using Surgeyor® nuclease assay. Red arrows indicate mutations.

FIG. ID shows plasma levels of FIX:C in the founder mice positive for the Surgeyor® nuclease assay.

FIG. IE shows the sequence of the F9 locus in two F2 male mice derived from a founder mouse.

FIGs. 2A to 2D show AAV vector-mediated generation of hemophilia B in adult wild-type mice.

FIG. 2A shows a schematic diagram of a single AAV vector expressing SaCas9 and sgRNA, where the HCRhAATp includes an enhancer element of the hepatic control region of the ApoE/C1 gene and the human anti-trypsin promoter.

FIG. 2B shows that the AAV vectors expressing SaCas9 and various sgRNAs targeting F9 were intravenously injected into 7-week old C57BL/6J male mice, and plasma levels of FIX:C were measured at the indicated times. Solid lines and dashed lines represent the high dose treatment (1×10¹² vg/body) and the low dose treatment (3×10¹¹ vg/body), respectively. The values are means ± SEM (n = 3 to 6). ** P < 0.01, as compared with the pretreatment (*post hoc* Bonferroni test).

FIG. 2C shows that Cas9-mediated cleavage of F9 in the liver was assessed by Surgeyor® nuclease assay. Control was liver DNA derived from non-treated C57BL/6Jb mice. Red arrows represent mutations.

FIG. 2D shows distribution of the AAV genome into each tissue at 8 weeks after the injection, where the values are means ± SEM (n = 6).

FIGs. 3A to 3C show marginal increase in FIX:C by HDR.

FIG. 3A shows a schematic diagram of the targeting strategy;

FIG. 3B shows that the AAV8 vector carrying SaCas9 and sgRNA targeting exon 8 of F9 (AAV8-SaCas9-sgRNA2 for F9 exon 8) as well as the AAV8 vector carrying the targeting sequence (AAV8-HDR Targeting) were injected intravenously into 2-week old (n = 4) or 26- to 45-week old (n = 5) hemophilia B mice generated by CRISPR/Cas9. Plasma FIX:C levels were measured at 8 weeks after the injection, where the vector doses, AAV8-sgRNA2/AAV8-HDR Targeting, were 2.4×10¹¹ vg/6×10¹¹ vg in 2-week old mice and 1×10¹² vg/3×10¹² vg in 26- to 45-week old mice. The values are means ± SEM. ** P < 0.01, as compared with the pretreatment (two-tailed student's t test).

FIG. 3C shows that Cas9-mediated cleavage of F9 in the liver was assessed using the Surgeyor® nuclease assay at 8 weeks after the injection. Control was liver DNA derived from non-treated hemophilia B mice generated by CRISPR/Cas9. Red arrows represent a mutation.

FIG. 4 shows a schematic diagram of targeting strategy. The AAV8 vector expressing SaCas9 and sgRNA targeting intron 1 of F9 (AAV8-SaCas9-sgRNA3 for F9 intron) induces a double-strand break (DSB) in intron 1. Simultaneous administration of the AAV8 vector and the cDNA of codon-optimized exon 2 to 8 of F9 (AAV8-Targeting) enabled correct insertion of the target sequence via the mechanisms of HDR and NHEJ, where SA: human F9 intron 1 splice acceptor site, F9 exon 2 to 8: codon-optimized cDNA (exon 2 to 8) of mouse F9, HDR: homologous directed repair, NHEJ: non-homologous end joining. Genotyping using primers (black small arrows) can distinguish HDR and NHEJ based on product sizes. Splicing after certain mRNA expression can be detected by the primers specific for codon-optimized F9 (red arrows).

FIGs. 5A to 5F show increases in plasma FIX:C in hemophilia B mice by CRIPSR/Ca9-mediated genome editing using an AAV8 vector.

FIG. 5A shows that AAV8 vector expressing SaCas9 and sgRNA targeting intron 1 of F9 (sgRNA3 for intron 1) (AAV8-SaCas9-sgRNA3 for F9 intron) was intravenously injected into 8-week old male C57BL/6J mice (1×10¹² vg/body), and Cas-mediated cleavage of the genome in the liver was verified by Surgeyor® nuclease assay. Control was liver DNA derived from non-treated C57BL/6J mice. Red arrows represent a mutation site.

FIG. 5B shows that hemophilia B male mice (4-week old) generated by the CRISPR/Cas9 system were treated with intravenous injection of AAV8-SaCas9-sgRNA3 for F9 intron (1×10¹² vg/body) and AAV8 vector carrying the target sequence (AAV8-Targeting: 3×10¹² vg/body). Plasma FIX:C levels were measured with one-stage clotting-time assay at the indicated times after the administration of the vector. The values are means ± SEM (n = 4). * P < 0.05, ** P < 0.01, as compared with the pretreatment (two-tailed student's t test).

FIG. 5C shows that liver genomic DNAs derived from hemophilia B mice non-treated (control) and treated (mice 1 and 2) with AAV8-SaCas9-sgRNA3 for F9 intron and AAV8-Targeting were subjected to PCR analysis to examine HDR and NHEJ at 6 weeks after the administration of the vector.

FIG. 5D shows that liver RNAs derived from non-treated mice (control) and hemophilia B mice (mice 1 and 2) treated with AAV8-SaCas9-sgRNA3 for F9 intron and AAV8-Targeting were subjected to RT-PCR to verify the expression of the codon-optimized F9 mRNA from the targeted genome sequences.

FIG. 5E shows that AAV8-SaCas9-sgRNA3 for F9 intron and AAV8-Targeting were injected into 0-day old (intraperitoneal injection, n = 6), 7-day old (intravenous injection, n = 6), 28-day old (intravenous injection, n = 6), and 42-day old (intravenous injection, n = 4) hemophilia B mice generated by CRISPR/Cas9. Plasma FIX:C levels were measured at 4 to 8 weeks after the injection. Vector doses, AAV8-SaCas9-sgRNA3 for F9 intron/AAV8-Targeting, were 6×10¹⁰vg/2×10¹¹ vg in 0-day old mice, 1.4 to 2.2×10¹¹ vg/4.1 to 6.6×10¹¹ vg in 7-day old mice, and 1×10¹² vg/3×10¹² vg in 28-day old and 42-day old mice. The values are means ± SEM. ** P < 0.01, as compared with the pretreatment (two-tailed student's t test).

FIG. 5F shows AAV genome in liver at 8 to 16 weeks after the injection of the vector. The values are means ± SEM (n = 3 to 5). **P < 0.01 (two-tailed student's test).

FIGs. 6A to 6F show restoring the bleeding phenotypes of hemophilia B mice by disruption of the AT gene, Serpinc1. AAV8 vector expressing SaCas9 and sgRNA targeting exon 8 of Serpinc1 (sgRNA1 for Serpinc1) was intravenously injected into 7- to 8-week old FIX-deficient hemophilia B male mice (1×10¹² vg/body).

FIG. 6A shows that plasma levels of AT:C were measured at the indicated times. The values are means ± SEM (n = 8). ** P < 0.01, as compared with the pretreatment (*post hoc* Bonferroni test).

FIG. 6B shows that Cas9-mediated cleavage in the liver was verified using Surveyor® nuclease assay. Red arrows indicate a mutation.

FIG. 6C shows that thrombin generation in plasma obtained at 4 to 8 weeks after the administration of the vector was assessed by the cleavage of fluorogenic substrate, and is expressed in arbitrary unit. The values are means ± SEM (n = 4). Thrombin generation in mouse plasma containing an indicated concentration of FIX:C (50%, 25%, 10%, 1%, and 0%) was assessed as reference. **P < 0.01 (two-tailed student's test).

FIGs. 6D and 6E show that fibrin formation *in vivo* after endothelial disruption in a wild-type mouse (C57BL/6), a FIX-deficient mouse (FIX-KO), and FIX-deficient mouse treated with an AAV8 vector targeting Serpinc1 (AT sgRNA) was observed by intravital confocal microscopy (Nikon A1RNP, Nikon Corporation) at 400× magnification. The scale bars represent 20 µm.

FIG. 6D shows representative images at 5 minutes after laser irradiation. The green signal indicates fibrin formation at the site of endothelial disruption (white arrow).

FIG. 6E shows that signal intensities of fibrin formation were quantified using NIS-Elements AR 3.2 (Nikon Corporation).

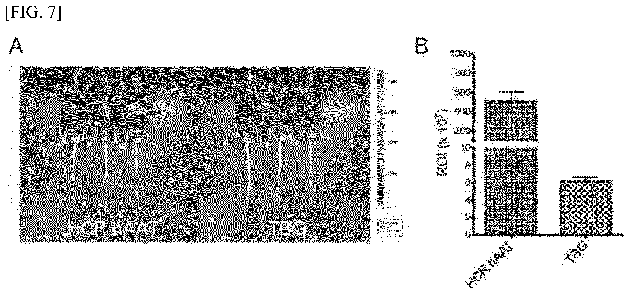

FIGs. 7A and 7B show that AAV8 vectors expressing luciferase downstream of HCRhAAT or TBG promoter were produced and were intravenously administered to male C57BL/6J mice at a dose of 1×10¹¹ vector genome/body. The luciferase activity after 2 weeks was detected with an IVIS imaging system (N = 3). The detection results demonstrated that the HCRhAAT promoter can express a reporter approximately 80 to 100 times that of the TBG promoter, and functions much more strongly than known liver-specific promoters used for Cas9 expression.

### 4. Summary

An AAV carrying Staphylococcus aureus Cas9 downstream of a liver-specific promoter and simultaneously expressing gRNA was produced, and it was succeeded to efficiently induce disruption of double-strand DNA in the coagulation factor/anticoagulation factor of liver genome. Since the coagulation factor level is decreased to 1% to 5% by disrupting factor IX which is a responsible gene for hemophilia B, mutations can be introduced into the genome of almost all hepatocytes. It was confirmed that the phenotype of hemophilia B can be improved by inserting cDNA into the first intron of F9 having a mutation using the present method. In addition, it was confirmed that the bleeding tendency of hemophilia can be improved by disrupting antithrombin, which suppresses blood coagulation.

### 5. References

Li, T. et. al., (2013) Mol Genet Genomic Med 1, 238-245.
Ran, F.A. et. al., (2015) Nature 520, 186-191.
Mimuro, J. et. al., (2013) the journal of the American Society of Gene Therapy 21, 318-323.
Matsumoto, T. et. al., (2009) Int J Hematol 90, 576-582.
Gao, G.P. et. al., (2002) Proceedings of the National Academy of Sciences of the United States of America 99, 11854-11859.
Nathwani, A.C. et. al., (2014) The New England journal of medicine 371, 1994-2004.
Nathwani, A.C. et. al., (2011) The New England journal of medicine 365, 2357-2365.
Wu, Y. et. al., (2016) Sci Rep 6, 18865.
Anguela, X.M. et. al., (2013) Blood 122, 3283-3287.
Sharma, R. et. al., (2015) Blood 126, 1777-1784.

### [Industrial Availability]

Europe and, in future, developing countries, rather than Japan, are expected as markets where the hemophilia gene therapy is used. Moreover, in future, the AAV vector according to the present invention may be of interest to companies as a new gene transfer tool using a genome editing technique.

### [Sequence Listing Free Text]

SEQ ID NO: 1: the nucleotide sequence of synthetic promoter/enhancer HCRhAAT
SEQ ID NO: 2: the amino acid sequence of the CRISPR/Cas9 protein
SEQ ID NO: 3: the nucleotide sequence of gRNA-1 (F9 exon 8) of CRISPR/Cas9
SEQ ID NO: 4: the nucleotide sequence of gRNA-2 (F9 exon 8) of CRISPR/Cas9
SEQ ID NO: 5: the nucleotide sequence of gRNA-3 (F9 exon 8) of CRISPR/Cas9
SEQ ID NO: 6: the amino acid sequence of the SaCas9 protein
SEQ ID NO: 7: the nucleotide sequence of gRNA-1 (F9 exon 8) of the SaCas9 protein
SEQ ID NO: 8: the nucleotide sequence of gRNA-2 (F9 exon 8) of the SaCas9 protein
SEQ ID NO: 9: the nucleotide sequence of gRNA-3 (F9 exon 8) of the SaCas9 protein
SEQ ID NO: 10: the nucleotide sequence of gRNA-1 (F9 intron 1) of the SaCas9 protein
SEQ ID NO: 11: the nucleotide sequence of gRNA-2 (F9 intron 1) of the SaCas9 protein
SEQ ID NO: 12: the nucleotide sequence of gRNA-3 (F9 intron 1) of the SaCas9 protein
SEQ ID NO: 13: the nucleotide sequence of gRNA-1 (Serpinc1 exon 8) of the SaCas9 protein
SEQ ID NO: 14: the nucleotide sequence of gRNA-2 (Serpinc1 exon 3) of the SaCas9 protein
SEQ ID NO: 15: the nucleotide sequence of gRNA-3 (Serpinc1 exon 3) of the SaCas9 protein
SEQ ID NO: 16: the repair gene (exon 2 to 8 + complementary region)
SEQ ID NO: 17: a primer for Surveyor assay (murine F9 exon 8F)
SEQ ID NO: 18: a primer for Surveyor assay (murine F9 exon 8R)
SEQ ID NO: 19: a primer for Surveyor assay (murine F9 intron 1F)
SEQ ID NO: 20: a primer for Surveyor assay (murine F9 intron 1R)
SEQ ID NO: 21: a primer for Surveyor assay (murine Serpinc1 exon 8F)
SEQ ID NO: 22: a primer for Surveyor assay (murine Serpinc2 exon 8R)
SEQ ID NO: 23: a primer for Surveyor assay (murine Serpinc1 exon 3F)
SEQ ID NO: 24: a primer for Surveyor assay (murine Serpinc1 exon 3R)
SEQ ID NO: 25: Primer F for real-time quantitative PCR
SEQ ID NO: 26: Primer R for real-time quantitative PCR
SEQ ID NO: 27: a primer probe for real-time quantitative PCR
SEQ ID NO: 28: a primer for deep sequencing (murine F9 exon 8F)
SEQ ID NO: 29: a primer for deep sequencing (murine F9 exon 8R)
SEQ ID NO: 30: a primer (F) for HDR/NHEJ detection
SEQ ID NO: 31: a primer (R) for HDR/NHEJ detection
SEQ ID NO: 32: a primer (F) for coF9 mRNA detection
SEQ ID NO: 33: a primer (R) for coF9 mRNA detection
SEQ ID NO: 34: TBG-HCR synthetic promoter sequence
SEQ ID NO: 35: Human SEPP1 promoter region (a liver-specific promoter)
SEQ ID NO: 36: Human TIE2 enhancer (an endothelial cell-specific enhancer)

## Claims

1. A recombinant adeno-associated virus vector for treating a blood coagulation-related disease,
wherein the virus vector comprises a viral genome comprising a liver-specific promoter sequence and a polynucleotide sequence encoding a genome editing means operably linked to the promoter sequence,
said genome editing means is
(a) a means comprising CRISPR/Cas9 composed of a Cas9 protein and a guide RNA (gRNA) and a repair gene, or
(b) a means comprising CRISPR/Cas9 composed of a Cas9 protein and a gRNA,
wherein the gRNA comprises a nucleotide region complementary to a part of a region related to expression of a disease-related protein on the genome of a patient and a region that interacts with the Cas9 protein.

2. The adeno-associated virus vector according to claim 1, wherein the vector comprises a capsid protein derived from an adeno-associated virus of AAV3, AAV3B, AAV8, or AAV9.

3. The recombinant adeno-associated virus vector according to claim 1 or 2, wherein the liver-specific promoter sequence comprises a polynucleotide having 90% or more homology with a polynucleotide sequence selected from the group consisting of ApoE promoter, anti-trypsin promoter, cKit promoter, a promoter of a liver-specific transcription factor (HNF-1, HNF-2, HNF-3, HNF-6, C/ERP, or DBP), a promoter of albumin or thyroxine binding globulin (TBG), and the polynucleotide sequence set forth in SEQ ID NO: 1, and functions as a liver-specific promoter.

4. The recombinant adeno-associated virus vector according to any one of claims 1 to 3, wherein the Cas9 protein comprises an amino acid sequence set forth in SEQ ID NO: 2 or 6 or comprises an amino acid sequence having 90% or more homology with an amino acid sequence set forth in SEQ ID NO: 2 or 6, and can be combined with a gRNA having a sequence of any of SEQ ID NOs: 3 to 5 or SEQ ID NOs: 7 to 15 to form a CRISPR/Cas9 complex.

5. The recombinant adeno-associated virus vector according to any one of claims 1 to 4, wherein the gRNA has a polynucleotide sequence set forth in any of SEQ ID NOs: 3 to 5 or SEQ ID NOs: 7 to 15, or comprises a polynucleotide sequence having 90% or more homology with a polynucleotide sequence set forth in any of SEQ ID NOs: 3 to 5 or SEQ ID NOs: 7 to 15 (3' sequence without crRNA and PAM portions), and can be combined with a Cas9 protein having a sequence set forth in SEQ ID NO: 2 or 6 to form a CRISPR/Cas9 complex.

6. The recombinant adeno-associated virus vector according to any one of claims 1 to 5, wherein the disease-related protein is a blood coagulation factor or an anticoagulant factor.

7. The recombinant adeno-associated virus vector according to claim 6, wherein the disease-related protein is blood coagulation factor IX, antitrypsin, blood coagulation factor VIII, blood coagulation factor VII, blood coagulation factor XI, antithrombin, protein S, or protein C.

8. The adeno-associated virus vector according to any one of claims 1 to 7, wherein the (a) comprises (a1) an adeno-associated virus vector encoding the CRISPR/Cas9 composed of the Cas9 protein and the gRNA and (a2) an adeno-associated virus vector comprising the repair gene.

9. The adeno-associated virus vector according to claim 8, wherein the (a2) comprises regions homologous to 5' and 3' sides of the cleavage target site in the genome on the 5' and 3' sides of the repair gene.

10. The adeno-associated virus vector according to claim 9, wherein the repair gene comprises a polynucleotide encoding a normal disease-related protein or a part thereof between the regions homologous to the 5' and 3' sides.

11. The recombinant adeno-associated virus vector according to any one of claims 1 to 10, wherein the blood coagulation-related disease is selected from the group consisting of hemophilia, factor VII deficiency, factor XI deficiency, antithrombin deficiency, protein S abnormality/deficiency, and protein C abnormality.

12. The recombinant adeno-associated virus vector according to any one of claims 1 to 11, wherein the virus genome further comprises a nucleotide sequence of an inverted terminal repeat (ITR) selected from the group consisting of AAV1, AAV2, AAV3, AAV4, AAV6, and AAV9.

13. A pharmaceutical composition for treating a liver-related disease, comprising the recombinant adeno-associated virus vector according to any one of claims 1 to 12.

14. The pharmaceutical composition according to claim 13, wherein the pharmaceutical composition comprises a combination of two or more recombinant adeno-associated virus vectors.

15. The pharmaceutical composition according to claim 13 or 14, wherein the pharmaceutical composition is used in combination with a therapeutic agent for hemophilia.

16. A medical kit for treating a liver-related disease, comprising the pharmaceutical composition according to any of claims 13 to 15.


SEQUENCE LISTING

<110>  Jichi Medical University

<120>  AAV vector for disruption of genes of coagulant relating factors
       in liver genome

<130>  G1657WO

<150>  JP 2017-004198
<151>  2017-01-13

<160>  36

<170>  PatentIn version 3.5

<210>  1
<211>  650
<212>  DNA
<213>  Artificial Sequence

<220>
<223>  HCRhAAT synthetic promoter

<400>  1
ctgcaggctc agaggcacac aggagtttct gggctcaccc tgcccccttc caacccctca      60

gttcccatcc tccagcagct gtttgtgtgc tgcctctgaa gtccacactg aacaaacttc     120

agcctactca tgtccctaaa atgggcaaac attgcaagca gcaaacagca aacacacagc     180

cctccctgcc tgctgacctt ggagctgggg cagaggtcag agacctctct gggcccatgc     240

cacctccaac atccactcga ccccttggaa tttcggtgga gaggagcaga ggttgtcctg     300

gcgtggttta ggtagtgtga gagggtccgg gttcaagggc gaattcgccc tcagagactg     360

tctgactcac gccaccccct ccaccttgga cacaggacgc tgtggtttct gagccaggta     420

caatgactcc tttcggtaag tgcagtggaa gctgtacact gcccaggcaa agcgtccggg     480

cagcgtaggc gggcgactca gatcccagcc agtggactta gcccctgttt gctcctccga     540

taactggggt gaccttggtt aatattcacc agcagcctcc cccgttgccc ctctggatcc     600

actgcttaaa tacggacgag gacagggccc tgtctcctca gcttcaggca                650


<210>  2
<211>  1409
<212>  PRT
<213>  Streptococcus pyogenes


<220>
<221>  MISC_FEATURE
<222>  (1)..(1409)
<223>  SpCas9 amino acid sequence

<400>  2

Met Gly Lys Pro Ile Pro Asn Pro Leu Leu Gly Leu Asp Ser Thr Ala
1               5                   10                  15

Pro Lys Lys Lys Arg Lys Val Gly Ile His Gly Val Pro Ala Ala Asp
            20                  25                  30

Lys Lys Tyr Ser Ile Gly Leu Asp Ile Gly Thr Asn Ser Val Gly Trp
        35                  40                  45

Ala Val Ile Thr Asp Glu Tyr Lys Val Pro Ser Lys Lys Phe Lys Val
    50                  55                  60

Leu Gly Asn Thr Asp Arg His Ser Ile Lys Lys Asn Leu Ile Gly Ala
65                  70                  75                  80

Leu Leu Phe Asp Ser Gly Glu Thr Ala Glu Ala Thr Arg Leu Lys Arg
                85                  90                  95

Thr Ala Arg Arg Arg Tyr Thr Arg Arg Lys Asn Arg Ile Cys Tyr Leu
            100                 105                 110

Gln Glu Ile Phe Ser Asn Glu Met Ala Lys Val Asp Asp Ser Phe Phe
        115                 120                 125

His Arg Leu Glu Glu Ser Phe Leu Val Glu Glu Asp Lys Lys His Glu
    130                 135                 140

Arg His Pro Ile Phe Gly Asn Ile Val Asp Glu Val Ala Tyr His Glu
145                 150                 155                 160

Lys Tyr Pro Thr Ile Tyr His Leu Arg Lys Lys Leu Val Asp Ser Thr
                165                 170                 175

Asp Lys Ala Asp Leu Arg Leu Ile Tyr Leu Ala Leu Ala His Met Ile
            180                 185                 190

Lys Phe Arg Gly His Phe Leu Ile Glu Gly Asp Leu Asn Pro Asp Asn
        195                 200                 205

Ser Asp Val Asp Lys Leu Phe Ile Gln Leu Val Gln Thr Tyr Asn Gln
    210                 215                 220

Leu Phe Glu Glu Asn Pro Ile Asn Ala Ser Gly Val Asp Ala Lys Ala
225                 230                 235                 240

Ile Leu Ser Ala Arg Leu Ser Lys Ser Arg Arg Leu Glu Asn Leu Ile
                245                 250                 255

Ala Gln Leu Pro Gly Glu Lys Lys Asn Gly Leu Phe Gly Asn Leu Ile

            260                 265                 270

Ala Leu Ser Leu Gly Leu Thr Pro Asn Phe Lys Ser Asn Phe Asp Leu
        275                 280                 285

Ala Glu Asp Ala Lys Leu Gln Leu Ser Lys Asp Thr Tyr Asp Asp Asp
    290                 295                 300

Leu Asp Asn Leu Leu Ala Gln Ile Gly Asp Gln Tyr Ala Asp Leu Phe
305                 310                 315                 320

Leu Ala Ala Lys Asn Leu Ser Asp Ala Ile Leu Leu Ser Asp Ile Leu
                325                 330                 335

Arg Val Asn Thr Glu Ile Thr Lys Ala Pro Leu Ser Ala Ser Met Ile
            340                 345                 350

Lys Arg Tyr Asp Glu His His Gln Asp Leu Thr Leu Leu Lys Ala Leu
        355                 360                 365

Val Arg Gln Gln Leu Pro Glu Lys Tyr Lys Glu Ile Phe Phe Asp Gln
    370                 375                 380

Ser Lys Asn Gly Tyr Ala Gly Tyr Ile Asp Gly Gly Ala Ser Gln Glu
385                 390                 395                 400

Glu Phe Tyr Lys Phe Ile Lys Pro Ile Leu Glu Lys Met Asp Gly Thr
                405                 410                 415

Glu Glu Leu Leu Val Lys Leu Asn Arg Glu Asp Leu Leu Arg Lys Gln
            420                 425                 430

Arg Thr Phe Asp Asn Gly Ser Ile Pro His Gln Ile His Leu Gly Glu
        435                 440                 445

Leu His Ala Ile Leu Arg Arg Gln Glu Asp Phe Tyr Pro Phe Leu Lys
    450                 455                 460

Asp Asn Arg Glu Lys Ile Glu Lys Ile Leu Thr Phe Arg Ile Pro Tyr
465                 470                 475                 480

Tyr Val Gly Pro Leu Ala Arg Gly Asn Ser Arg Phe Ala Trp Met Thr
                485                 490                 495

Arg Lys Ser Glu Glu Thr Ile Thr Pro Trp Asn Phe Glu Glu Val Val
            500                 505                 510

Asp Lys Gly Ala Ser Ala Gln Ser Phe Ile Glu Arg Met Thr Asn Phe
        515                 520                 525

Asp Lys Asn Leu Pro Asn Glu Lys Val Leu Pro Lys His Ser Leu Leu
    530                 535                 540

Tyr Glu Tyr Phe Thr Val Tyr Asn Glu Leu Thr Lys Val Lys Tyr Val
545                 550                 555                 560

Thr Glu Gly Met Arg Lys Pro Ala Phe Leu Ser Gly Glu Gln Lys Lys
                565                 570                 575

Ala Ile Val Asp Leu Leu Phe Lys Thr Asn Arg Lys Val Thr Val Lys
            580                 585                 590

Gln Leu Lys Glu Asp Tyr Phe Lys Lys Ile Glu Cys Phe Asp Ser Val
        595                 600                 605

Glu Ile Ser Gly Val Glu Asp Arg Phe Asn Ala Ser Leu Gly Thr Tyr
    610                 615                 620

His Asp Leu Leu Lys Ile Ile Lys Asp Lys Asp Phe Leu Asp Asn Glu
625                 630                 635                 640

Glu Asn Glu Asp Ile Leu Glu Asp Ile Val Leu Thr Leu Thr Leu Phe
                645                 650                 655

Glu Asp Arg Glu Met Ile Glu Glu Arg Leu Lys Thr Tyr Ala His Leu
            660                 665                 670

Phe Asp Asp Lys Val Met Lys Gln Leu Lys Arg Arg Arg Tyr Thr Gly
        675                 680                 685

Trp Gly Arg Leu Ser Arg Lys Leu Ile Asn Gly Ile Arg Asp Lys Gln
    690                 695                 700

Ser Gly Lys Thr Ile Leu Asp Phe Leu Lys Ser Asp Gly Phe Ala Asn
705                 710                 715                 720

Arg Asn Phe Met Gln Leu Ile His Asp Asp Ser Leu Thr Phe Lys Glu
                725                 730                 735

Asp Ile Gln Lys Ala Gln Val Ser Gly Gln Gly Asp Ser Leu His Glu
            740                 745                 750

His Ile Ala Asn Leu Ala Gly Ser Pro Ala Ile Lys Lys Gly Ile Leu
        755                 760                 765

Gln Thr Val Lys Val Val Asp Glu Leu Val Lys Val Met Gly Arg His
    770                 775                 780

Lys Pro Glu Asn Ile Val Ile Glu Met Ala Arg Glu Asn Gln Thr Thr
785                 790                 795                 800

Gln Lys Gly Gln Lys Asn Ser Arg Glu Arg Met Lys Arg Ile Glu Glu
                805                 810                 815

Gly Ile Lys Glu Leu Gly Ser Gln Ile Leu Lys Glu His Pro Val Glu
            820                 825                 830

Asn Thr Gln Leu Gln Asn Glu Lys Leu Tyr Leu Tyr Tyr Leu Gln Asn
        835                 840                 845

Gly Arg Asp Met Tyr Val Asp Gln Glu Leu Asp Ile Asn Arg Leu Ser
    850                 855                 860

Asp Tyr Asp Val Asp His Ile Val Pro Gln Ser Phe Leu Lys Asp Asp
865                 870                 875                 880

Ser Ile Asp Asn Lys Val Leu Thr Arg Ser Asp Lys Asn Arg Gly Lys
                885                 890                 895

Ser Asp Asn Val Pro Ser Glu Glu Val Val Lys Lys Met Lys Asn Tyr
            900                 905                 910

Trp Arg Gln Leu Leu Asn Ala Lys Leu Ile Thr Gln Arg Lys Phe Asp
        915                 920                 925

Asn Leu Thr Lys Ala Glu Arg Gly Gly Leu Ser Glu Leu Asp Lys Ala
    930                 935                 940

Gly Phe Ile Lys Arg Gln Leu Val Glu Thr Arg Gln Ile Thr Lys His
945                 950                 955                 960

Val Ala Gln Ile Leu Asp Ser Arg Met Asn Thr Lys Tyr Asp Glu Asn
                965                 970                 975

Asp Lys Leu Ile Arg Glu Val Lys Val Ile Thr Leu Lys Ser Lys Leu
            980                 985                 990

Val Ser Asp Phe Arg Lys Asp Phe  Gln Phe Tyr Lys Val  Arg Glu Ile
        995                 1000                 1005

Asn Asn  Tyr His His Ala His  Asp Ala Tyr Leu Asn  Ala Val Val
    1010                 1015                 1020

Gly Thr Ala Leu Ile Lys Lys Tyr Pro Lys Leu Glu Ser Glu Phe
    1025                1030                1035

Val Tyr Gly Asp Tyr Lys Val Tyr Asp Val Arg Lys Met Ile Ala
    1040                1045                1050

Lys Ser Glu Gln Glu Ile Gly Lys Ala Thr Ala Lys Tyr Phe Phe
    1055                1060                1065

Tyr Ser Asn Ile Met Asn Phe Phe Lys Thr Glu Ile Thr Leu Ala
    1070                1075                1080

Asn Gly Glu Ile Arg Lys Arg Pro Leu Ile Glu Thr Asn Gly Glu
    1085                1090                1095

Thr Gly Glu Ile Val Trp Asp Lys Gly Arg Asp Phe Ala Thr Val
    1100                1105                1110

Arg Lys Val Leu Ser Met Pro Gln Val Asn Ile Val Lys Lys Thr
    1115                1120                1125

Glu Val Gln Thr Gly Gly Phe Ser Lys Glu Ser Ile Leu Pro Lys
    1130                1135                1140

Arg Asn Ser Asp Lys Leu Ile Ala Arg Lys Lys Asp Trp Asp Pro
    1145                1150                1155

Lys Lys Tyr Gly Gly Phe Asp Ser Pro Thr Val Ala Tyr Ser Val
    1160                1165                1170

Leu Val Val Ala Lys Val Glu Lys Gly Lys Ser Lys Lys Leu Lys
    1175                1180                1185

Ser Val Lys Glu Leu Leu Gly Ile Thr Ile Met Glu Arg Ser Ser
    1190                1195                1200

Phe Glu Lys Asn Pro Ile Asp Phe Leu Glu Ala Lys Gly Tyr Lys
    1205                1210                1215

Glu Val Lys Lys Asp Leu Ile Ile Lys Leu Pro Lys Tyr Ser Leu
    1220                1225                1230

Phe Glu Leu Glu Asn Gly Arg Lys Arg Met Leu Ala Ser Ala Gly
    1235                1240                1245

Glu Leu Gln Lys Gly Asn Glu Leu Ala Leu Pro Ser Lys Tyr Val

1250                    1255                    1260

Asn Phe Leu Tyr Leu Ala Ser His Tyr Glu Lys Leu Lys Gly Ser
    1265                    1270                    1275

Pro Glu Asp Asn Glu Gln Lys Gln Leu Phe Val Glu Gln His Lys
    1280                    1285                    1290

His Tyr Leu Asp Glu Ile Ile Glu Gln Ile Ser Glu Phe Ser Lys
    1295                    1300                    1305

Arg Val Ile Leu Ala Asp Ala Asn Leu Asp Lys Val Leu Ser Ala
    1310                    1315                    1320

Tyr Asn Lys His Arg Asp Lys Pro Ile Arg Glu Gln Ala Glu Asn
    1325                    1330                    1335

Ile Ile His Leu Phe Thr Leu Thr Asn Leu Gly Ala Pro Ala Ala
    1340                    1345                    1350

Phe Lys Tyr Phe Asp Thr Thr Ile Asp Arg Lys Arg Tyr Thr Ser
    1355                    1360                    1365

Thr Lys Glu Val Leu Asp Ala Thr Leu Ile His Gln Ser Ile Thr
    1370                    1375                    1380

Gly Leu Tyr Glu Thr Arg Ile Asp Leu Ser Gln Leu Gly Gly Asp
    1385                    1390                    1395

Ser Arg Ala Asp Pro Lys Lys Lys Arg Lys Val
    1400                    1405

<210>  3
<211>  101
<212>  DNA
<213>  Artificial Sequence

<220>
<223>  gRNA1 (F9 exon8) for SpCas9

<400>  3
gtcttcaaca aagggagacg ttttagagct agaaatagca agttaaaata aggctagtcc      60

gttatcaact tgaaaaagtg gcaccgagtc ggtgcttttt t                          101

<210>  4
<211>  101
<212>  DNA
<213>  Artificial Sequence

<220>

<223>  gRNA2 (F9 exon8) for SpCas9

<400>  4
cagtacctta gagttccacg ttttagagct agaaatagca agttaaaata aggctagtcc      60

gttatcaact tgaaaaagtg gcaccgagtc ggtgcttttt t                          101


<210>  5
<211>  95
<212>  DNA
<213>  Artificial Sequence

<220>
<223>  gRNA3 (F9 exon8) for SpCas9

<400>  5
ggtttcccgg tacgtcaacg ttttagagct agaaatagca agttaaaata aggctagtcc      60

gttatcaact tgaaaaagtg gcaccgagtc ggtgc                                 95


<210>  6
<211>  1053
<212>  PRT
<213>  Staphylococcus aureus


<220>
<221>  MISC_FEATURE
<222>  (1)..(1053)
<223>  SaCas9 amino acid sequence

<400>  6

Met Lys Arg Asn Tyr Ile Leu Gly Leu Asp Ile Gly Ile Thr Ser Val
1               5                   10                  15


Gly Tyr Gly Ile Ile Asp Tyr Glu Thr Arg Asp Val Ile Asp Ala Gly
            20                  25                  30


Val Arg Leu Phe Lys Glu Ala Asn Val Glu Asn Asn Glu Gly Arg Arg
        35                  40                  45


Ser Lys Arg Gly Ala Arg Arg Leu Lys Arg Arg Arg Arg His Arg Ile
    50                  55                  60


Gln Arg Val Lys Lys Leu Leu Phe Asp Tyr Asn Leu Leu Thr Asp His
65                  70                  75                  80


Ser Glu Leu Ser Gly Ile Asn Pro Tyr Glu Ala Arg Val Lys Gly Leu
                85                  90                  95


Ser Gln Lys Leu Ser Glu Glu Glu Phe Ser Ala Ala Leu Leu His Leu
            100                 105                 110

Ala Lys Arg Arg Gly Val His Asn Val Asn Glu Val Glu Glu Asp Thr
        115                 120                 125

Gly Asn Glu Leu Ser Thr Lys Glu Gln Ile Ser Arg Asn Ser Lys Ala
    130                 135                 140

Leu Glu Glu Lys Tyr Val Ala Glu Leu Gln Leu Glu Arg Leu Lys Lys
145                 150                 155                 160

Asp Gly Glu Val Arg Gly Ser Ile Asn Arg Phe Lys Thr Ser Asp Tyr
                165                 170                 175

Val Lys Glu Ala Lys Gln Leu Leu Lys Val Gln Lys Ala Tyr His Gln
            180                 185                 190

Leu Asp Gln Ser Phe Ile Asp Thr Tyr Ile Asp Leu Leu Glu Thr Arg
        195                 200                 205

Arg Thr Tyr Tyr Glu Gly Pro Gly Glu Gly Ser Pro Phe Gly Trp Lys
    210                 215                 220

Asp Ile Lys Glu Trp Tyr Glu Met Leu Met Gly His Cys Thr Tyr Phe
225                 230                 235                 240

Pro Glu Glu Leu Arg Ser Val Lys Tyr Ala Tyr Asn Ala Asp Leu Tyr
                245                 250                 255

Asn Ala Leu Asn Asp Leu Asn Asn Leu Val Ile Thr Arg Asp Glu Asn
            260                 265                 270

Glu Lys Leu Glu Tyr Tyr Glu Lys Phe Gln Ile Ile Glu Asn Val Phe
        275                 280                 285

Lys Gln Lys Lys Lys Pro Thr Leu Lys Gln Ile Ala Lys Glu Ile Leu
    290                 295                 300

Val Asn Glu Glu Asp Ile Lys Gly Tyr Arg Val Thr Ser Thr Gly Lys
305                 310                 315                 320

Pro Glu Phe Thr Asn Leu Lys Val Tyr His Asp Ile Lys Asp Ile Thr
                325                 330                 335

Ala Arg Lys Glu Ile Ile Glu Asn Ala Glu Leu Leu Asp Gln Ile Ala
            340                 345                 350

Lys Ile Leu Thr Ile Tyr Gln Ser Ser Glu Asp Ile Gln Glu Glu Leu
        355                 360                 365

Thr Asn Leu Asn Ser Glu Leu Thr Gln Glu Glu Ile Glu Gln Ile Ser
    370                 375                 380

Asn Leu Lys Gly Tyr Thr Gly Thr His Asn Leu Ser Leu Lys Ala Ile
385                 390                 395                 400

Asn Leu Ile Leu Asp Glu Leu Trp His Thr Asn Asp Asn Gln Ile Ala
                405                 410                 415

Ile Phe Asn Arg Leu Lys Leu Val Pro Lys Lys Val Asp Leu Ser Gln
            420                 425                 430

Gln Lys Glu Ile Pro Thr Thr Leu Val Asp Asp Phe Ile Leu Ser Pro
        435                 440                 445

Val Val Lys Arg Ser Phe Ile Gln Ser Ile Lys Val Ile Asn Ala Ile
    450                 455                 460

Ile Lys Lys Tyr Gly Leu Pro Asn Asp Ile Ile Ile Glu Leu Ala Arg
465                 470                 475                 480

Glu Lys Asn Ser Lys Asp Ala Gln Lys Met Ile Asn Glu Met Gln Lys
                485                 490                 495

Arg Asn Arg Gln Thr Asn Glu Arg Ile Glu Glu Ile Ile Arg Thr Thr
            500                 505                 510

Gly Lys Glu Asn Ala Lys Tyr Leu Ile Glu Lys Ile Lys Leu His Asp
        515                 520                 525

Met Gln Glu Gly Lys Cys Leu Tyr Ser Leu Glu Ala Ile Pro Leu Glu
    530                 535                 540

Asp Leu Leu Asn Asn Pro Phe Asn Tyr Glu Val Asp His Ile Ile Pro
545                 550                 555                 560

Arg Ser Val Ser Phe Asp Asn Ser Phe Asn Asn Lys Val Leu Val Lys
                565                 570                 575

Gln Glu Glu Asn Ser Lys Lys Gly Asn Arg Thr Pro Phe Gln Tyr Leu
            580                 585                 590

Ser Ser Ser Asp Ser Lys Ile Ser Tyr Glu Thr Phe Lys Lys His Ile
        595                 600                 605

Leu Asn Leu Ala Lys Gly Lys Gly Arg Ile Ser Lys Thr Lys Lys Glu
    610                 615                 620

Tyr Leu Leu Glu Glu Arg Asp Ile Asn Arg Phe Ser Val Gln Lys Asp
625                 630                 635                 640

Phe Ile Asn Arg Asn Leu Val Asp Thr Arg Tyr Ala Thr Arg Gly Leu
                645                 650                 655

Met Asn Leu Leu Arg Ser Tyr Phe Arg Val Asn Asn Leu Asp Val Lys
            660                 665                 670

Val Lys Ser Ile Asn Gly Gly Phe Thr Ser Phe Leu Arg Arg Lys Trp
        675                 680                 685

Lys Phe Lys Lys Glu Arg Asn Lys Gly Tyr Lys His His Ala Glu Asp
    690                 695                 700

Ala Leu Ile Ile Ala Asn Ala Asp Phe Ile Phe Lys Glu Trp Lys Lys
705                 710                 715                 720

Leu Asp Lys Ala Lys Lys Val Met Glu Asn Gln Met Phe Glu Glu Lys
                725                 730                 735

Gln Ala Glu Ser Met Pro Glu Ile Glu Thr Glu Gln Glu Tyr Lys Glu
            740                 745                 750

Ile Phe Ile Thr Pro His Gln Ile Lys His Ile Lys Asp Phe Lys Asp
        755                 760                 765

Tyr Lys Tyr Ser His Arg Val Asp Lys Lys Pro Asn Arg Glu Leu Ile
    770                 775                 780

Asn Asp Thr Leu Tyr Ser Thr Arg Lys Asp Asp Lys Gly Asn Thr Leu
785                 790                 795                 800

Ile Val Asn Asn Leu Asn Gly Leu Tyr Asp Lys Asp Asn Asp Lys Leu
                805                 810                 815

Lys Lys Leu Ile Asn Lys Ser Pro Glu Lys Leu Leu Met Tyr His His
            820                 825                 830

Asp Pro Gln Thr Tyr Gln Lys Leu Lys Leu Ile Met Glu Gln Tyr Gly
        835                 840                 845

Asp Glu Lys Asn Pro Leu Tyr Lys Tyr Tyr Glu Glu Thr Gly Asn Tyr
    850                 855                 860

Leu Thr Lys Tyr Ser Lys Lys Asp Asn Gly Pro Val Ile Lys Lys Ile

865                 870                 875                 880

Lys Tyr Tyr Gly Asn Lys Leu Asn Ala His Leu Asp Ile Thr Asp Asp
                885                 890                 895

Tyr Pro Asn Ser Arg Asn Lys Val Val Lys Leu Ser Leu Lys Pro Tyr
            900                 905                 910

Arg Phe Asp Val Tyr Leu Asp Asn Gly Val Tyr Lys Phe Val Thr Val
        915                 920                 925

Lys Asn Leu Asp Val Ile Lys Lys Glu Asn Tyr Tyr Glu Val Asn Ser
    930                 935                 940

Lys Cys Tyr Glu Glu Ala Lys Lys Leu Lys Lys Ile Ser Asn Gln Ala
945                 950                 955                 960

Glu Phe Ile Ala Ser Phe Tyr Asn Asn Asp Leu Ile Lys Ile Asn Gly
                965                 970                 975

Glu Leu Tyr Arg Val Ile Gly Val Asn Asn Asp Leu Leu Asn Arg Ile
            980                 985                 990

Glu Val Asn Met Ile Asp Ile Thr  Tyr Arg Glu Tyr Leu  Glu Asn Met
        995                 1000                1005

Asn Asp  Lys Arg Pro Pro Arg  Ile Ile Lys Thr Ile  Ala Ser Lys
    1010                1015                1020

Thr Gln  Ser Ile Lys Lys Tyr  Ser Thr Asp Ile Leu  Gly Asn Leu
    1025                1030                1035

Tyr Glu  Val Lys Ser Lys Lys  His Pro Gln Ile Ile  Lys Lys Gly
    1040                1045                1050

<210>  7
<211>  105
<212>  DNA
<213>  Artificial Sequence

<220>
<223>  gRNA1 (F9 exon8) for SaCas9

<400>  7
tcaacaaagg gagacaggct tgttttagta ctctggaaac agaatctact aaaacaaggc      60

aaaatgccgt gtttatctcg tcaacttgtt ggcgagattt ttttt                     105

<210>  8
<211>  105

<212>  DNA
<213>  Artificial Sequence

<220>
<223>  gRNA2 (F9 exon8) for SaCas9

<400>  8
cagtacctta gagttccact ggttttagta ctctggaaac agaatctact aaaacaaggc      60

aaaatgccgt gtttatctcg tcaacttgtt ggcgagattt ttttt                     105


<210>  9
<211>  105
<212>  DNA
<213>  Artificial Sequence

<220>
<223>  gRNA3 (F9 exon8) for SaCas9

<400>  9
taaggtttcc cggtacgtca agttttagta ctctggaaac agaatctact aaaacaaggc      60

aaaatgccgt gtttatctcg tcaacttgtt ggcgagattt ttttt                     105


<210>  10
<211>  105
<212>  DNA
<213>  Artificial Sequence

<220>
<223>  gRNA1 (F9 intron1) for SaCas9

<400>  10
ttgatcccga gggtctatac agttttagta ctctggaaac agaatctact aaaacaaggc      60

aaaatgccgt gtttatctcg tcaacttgtt ggcgagattt ttttt                     105


<210>  11
<211>  105
<212>  DNA
<213>  Artificial Sequence

<220>
<223>  gRNA2 (F9 intron1) for SaCas9

<400>  11
caggagacca gccgattttc tgttttagta ctctggaaac agaatctact aaaacaaggc      60

aaaatgccgt gtttatctcg tcaacttgtt ggcgagattt ttttt                     105


<210>  12
<211>  105
<212>  DNA
<213>  Artificial Sequence

<220>
<223>  gRNA3 (F9 intron1) for SaCas9

<400>  12

tccctcacca ctaagacgtg cgttttagta ctctggaaac agaatctact aaaacaaggc 60

aaaatgccgt gtttatctcg tcaacttgtt ggcgagattt ttttt 105

<210> 13
<211> 105
<212> DNA
<213> Artificial Sequence

<220>
<223> gRNA1 (Serpinc1 exon8) for SaCas9

<400> 13
gaggaaggca gtgaagcagc agttttagta ctctggaaac agaatctact aaaacaaggc 60

aaaatgccgt gtttatctcg tcaacttgtt ggcgagattt ttttt 105

<210> 14
<211> 105
<212> DNA
<213> Artificial Sequence

<220>
<223> gRNA2 (Serpinc1 exon8) for SaCas9

<400> 14
cctgggaaga aggccaccga ggttttagta ctctggaaac agaatctact aaaacaaggc 60

aaaatgccgt gtttatctcg tcaacttgtt ggcgagattt ttttt 105

<210> 15
<211> 105
<212> DNA
<213> Artificial Sequence

<220>
<223> gRNA3 (Serpinc1 exon8) for SaCas9

<400> 15
actgcttttg ctatgaccaa ggttttagta ctctggaaac agaatctact aaaacaaggc 60

aaaatgccgt gtttatctcg tcaacttgtt ggcgagattt ttttt 105

<210> 16
<211> 3650
<212> DNA
<213> Artificial Sequence

<220>
<223> Repair gene having 5′ arm-human FIX intron 1 (partial),-
codon-optimized mouse FIX cDNA (exon 2-8) and SV40 polyA-3′ arm

<220>
<221> 5′UTR
<222> (1)..(1027)
<223> 5′ arm

<220>

<221>  Intron
<222>  (1028)..(1182)
<223>  human FIX intron 1 (partial)

<220>
<221>  misc_feature
<222>  (1183)..(2510)
<223>  codon-optimized mouse FIX cDNA (exon 2-8)

<220>
<221>  3'UTR
<222>  (2511)..(2645)
<223>  SV40 polyA

<220>
<221>  3'UTR
<222>  (2646)..(3650)
<223>  SV40 polyA-3' arm

<400>  16
ggccgctctg tggtaacttc agtttcaata tttgaaaaaa cgaactacat ttacagggct      60

tgtggtgcat ttttatttaa tagatacagt tattcttatg atttgacaat ataattccac     120

ttaaaattta tgtgtccttg gttttaaaaa aatgaaaatc ggaaaatgtc ttaacaaaga     180

aattgcacaa taagcaggaa acatgttatt ttaggatttt agactcatca ccttgaaata     240

aaaaggggaa acatcaaaac cacaatggtt aatttgcata aaaagtcttt cttgaatcta     300

catgtgttcc agtgttttag aactagatgg taaggcatag ttataatgtt taaacatata     360

tttatagttt aatttctcag ctaaaattga aagattacgc ttttattact aagttgctgt     420

ctgtaatgaa agcttttaca tagatggaaa taaatcagct catctgtcat acatctcaga     480

gctgatgttt agagaaccat aatactcaga ggcctcccta ggggacaaaa gtaagtatac     540

aaatggccag aaagaggagt agcttaattc atgcaaagta cagcacatca gggtttcgtt     600

tggtttcgtt ttttgcaaca acagtggcat tactccccag ggaaaggcac acaggaagaa     660

cttactggct ttagacaaac agcctgattg ccccagatgc atttatcaca cttactagtc     720

aacctactaa agtcaagcaa gtgacagtgc caggatcaga ggtgccaacc ctgagcatcc     780

ccagaaaatc ggctggtctc ctggttccca ctccagacat gatgtcaact gtggaatcag     840

acttaaaagc tgaaatgatg atgtgagaat gcaaaggtta aactgcacta gcaaagtggg     900

tagagatgaa aaatacatgt tggaaactca ttacgtggac cataaaggca ggtcattcaa     960

cctagtgcat cagattgacc tatgtgagaa caaaagatcc aagcactctc ttataaagat    1020

ccaagttaac gtgggaacat cacagatttt ggctccatgc cctaaagaga aattggcttt    1080

cagattattt ggattaaaaa caaagacttt cttaagagat gtaaaatttt catgatgttt    1140

tcttttttgc taaaactaaa gaattattct tttacatttc agttttcctg gatagggaaa    1200

acgcaactaa gatcctgacc aggccaaaac gctataacag cggaaagctg gaagagtttg    1260

tgaggggcaa tctggaacga gaatgtattg aggaaaggtg ctcatttgaa gaggcccgag    1320

aggtgtttga aaatactgaa aagacaaccg agttctggaa acagtacgtg gatggggacc 1380

agtgcgagag caacccctgc ctgaacggcg gaatctgcaa ggatgacatc agctcatacg 1440

aatgctggtg tcaggtcggc tttgagggaa ggaactgtga gctggacgct acatgcaaca 1500

tcaagaatgg aaggtgcaag cagttctgta aaaacagccc cgacaacaaa gtcatctgca 1560

gctgcacaga agggtatcag ctggctgagg atcagaaatc ctgcgaacct accgtcccat 1620

ttccctgtgg gagagcttcc atcagctatt ccagcaagaa aatcacaagg gccgagacag 1680

tgtttagcaa catggactac gagaacagca cagaggccgt gttcattcag gacgatatta 1740

ctgacggagc cattctgaac aatgtgacag agagtagcga atccctgaat gatttcacaa 1800

gggtcgtggg cggagaaaac gctaaaccag ggcagattcc ctggcaggtc attctgaacg 1860

gagaaattga ggctttctgc ggcggggcca tcattaacga gaaatggatc gtgactgcag 1920

cccactgcct gaaaccaggc gacaagatcg aggtggtggc tggcgaatat aacatcgaca 1980

agaaagagga tacagagcag agaaggaacg tgattcggac aatcccacat caccagtaca 2040

acgcaacaat caacaagtac agtcacgaca tcgcactgct ggagctggac aagcctctga 2100

tcctgaactc ctacgtgaca ccaatctgcg tcgcaaaccg ggaatatacc aacattttcc 2160

tgaaattcgg gtccggatac gtgtccgggt ggggaaaggt cttcaacaaa gggaggcagg 2220

cctcaatcct gcagtatctg cgagtccccc tggtggatag ggccacatgc ctgcggagca 2280

ctaccttcac tatctacaat aacatgttct gcgccggata tagagaagga ggcaaagata 2340

gctgcgaggg agattcagga ggcccacatg tgaccgaagt cgaggggaca tcatttctga 2400

ccgggattat ttcatgggga gaggaatgtg caatgaaagg gaaatatggg atctacacca 2460

aagtgagcag atacgtgaac tggattaagg aaaaaacaaa actgacataa aacttgttta 2520

ttgcagctta taatggttac aaataaagca atagcatcac aaatttcaca aataaagcat 2580

ttttttcact gcattctagt tgtggtttgt ccaaactcat caatgtatct tatcatgtct 2640

ggatcccggt atctcagatg taaaatgata gaaaaaaatc atgatttcag agctgtggca 2700

ataatgacta aagaggtgtt aaatatatga tccccagaca gtattgagga taaaaagaac 2760

aagattgtga gtcagtttgg tcagggaacc agggaaatgg ccctggctgg ggagtggggc 2820

taggtattca taaagcacac tcttctgtta aacggtcctg ggctttagaa cagagaggcc 2880

ccatgttcat aactactgcc ttattgtgac aaattagaat tgggaaatca aaattagtct 2940

gtgaaatgaa tttgtgctta caatgtatgc aaatcaaaat tatctttaat gcatgtattt 3000

ggatattagt ccatactgtt gtggttcgtg gtactgtgac aaggtactgt ggcagagatg 3060

aatgggttca gaaaaaaaga ctttaatggg aagggatgag gtgtgtacca catgcttagg 3120

aagtacaaaa gaaggaacat aatggatgta aaatctcctg aaagatacag tgtttggagc 3180

aagatctcaa gaaatgaata gttgcccact atccttaaat gtctgacctg gatggttcat 3240

tgggcaaaac tgctttccac caagcctaac aacctgagtt tgatcccgag ggtctataca 3300

gtgaataaga gacttgactt ccaggattgt catttaatcc ccacatgtat gagaacactt 3360

acaatgcatg tatgcataaa tacagatgga tgtatataca tgtatgtgta tatgtaaaca 3420

cacacacata ttcttagggt ttatgtagtc attttaaaca ccatgaccat aagtaacttg 3480

gggcgaaaag gtttatttca gctgggggaa gtcagagagg gaactgaaac cagacaagaa 3540

cctggaggca ggagctgata tagaagccat ggagaaatac tgcttactgt cttcctcctc 3600

aaggcttgct ccgactgctt tcttacacaa ctcagaaaca ggatcaccgc 3650

<210> 17
<211> 19
<212> DNA
<213> Artificial Sequence

<220>
<223> Exon 8 of mouse F9 (forward) for Surveyor Assay

<400> 17
aactgggcaa atgggagag 19

<210> 18
<211> 19
<212> DNA
<213> Artificial Sequence

<220>
<223> Exon 8 of mouse F9 (reverse) for Surveyor Assay

<400> 18
tcaggagagg aagtcatgc 19

<210> 19
<211> 20
<212> DNA
<213> Artificial Sequence

<220>
<223> Intron 1 of mouse F9 (forward) for Surveyor Assay

<400> 19
aacagtggca ttactcccca 20

<210> 20
<211> 20
<212> DNA
<213> Artificial Sequence

<220>
<223> Intron 1 of mouse F9 (reverse) for Surveyor Assay

<400> 20
ccaaactggc ctgtgagaaa 20

<210>  21
<211>  20
<212>  DNA
<213>  Artificial Sequence

<220>
<223>  Exon 8 of mouse Serpinc1 (forward) for Surveyor Assay

<400>  21
gtggtagtga tagctgggat                                                   20


<210>  22
<211>  20
<212>  DNA
<213>  Artificial Sequence

<220>
<223>  Exon 8 of mouse Serpinc1 (reverse) for Surveyor Assay

<400>  22
gggagattga ttcgggtttg                                                   20


<210>  23
<211>  20
<212>  DNA
<213>  Artificial Sequence

<220>
<223>  Exon 3 of mouse Serpinc1 (forward) for Surveyor Assay

<400>  23
ccttgtctgg ggttttctga                                                   20


<210>  24
<211>  20
<212>  DNA
<213>  Artificial Sequence

<220>
<223>  Exon 3 of mouse Serpinc1 (reverse) for Surveyor Assay

<400>  24
gatcactggg tgtctttcca                                                   20


<210>  25
<211>  25
<212>  DNA
<213>  Artificial Sequence

<220>
<223>  Real time qPCR SV40 polyA (forward)

<400>  25
agcaatagca tcacaaattt cacaa                                             25


<210>  26

<211>  29
<212>  DNA
<213>  Artificial Sequence

<220>
<223>  SV40 polyA (reverse) for real time qPCR

<400>  26
ccagacatga taagatacat tgatgagtt                                          29


<210>  27
<211>  36
<212>  DNA
<213>  Artificial Sequence

<220>
<223>  SV40 polyA (prove) for real time qPCR

<400>  27
agcatttttt tcactgcatt ctagttgtgg tttgtc                                  36


<210>  28
<211>  59
<212>  DNA
<213>  Artificial Sequence

<220>
<223>  Exon 8 of mouse F9 (forward) for deep sequencing

<400>  28
tcgtcggcag cgtcagatgt gtataagaga cagtgatcag tgaagccaac cagactggg         59


<210>  29
<211>  60
<212>  DNA
<213>  Artificial Sequence

<220>
<223>  Exon 8 of mouse F9 (reverse) for deep sequencing

<400>  29
gtctcgtggg ctcggagatg tgtataagag acagccttca cacgaatctt tgcctccttc        60


<210>  30
<211>  20
<212>  DNA
<213>  Artificial Sequence

<220>
<223>  HDR and NHEJ Detection primer (forward)

<400>  30
gggatctaca ccaaagtgag                                                    20


<210>  31
<211>  20
<212>  DNA
<213>  Artificial Sequence

<220>
<223>  HDR and NHEJ detection primer (reverse)

<400>  31
ccaaactggc ctgtgagaaa                                                   20


<210>  32
<211>  20
<212>  DNA
<213>  Artificial Sequence

<220>
<223>  coF9 mRNA Detection primer (forward)

<400>  32
atgaagcacc tgaacaccgt                                                   20


<210>  33
<211>  20
<212>  DNA
<213>  Artificial Sequence

<220>
<223>  coF9 mRNA Detection primer (reverse)

<400>  33
ccagttcacg tatctgctca                                                   20


<210>  34
<211>  460
<212>  DNA
<213>  Homo sapiens


<220>
<221>  promoter
<222>  (1)..(460)
<223>  human TBG promoter

<400>  34
gggctggaag ctacctttga catcatttcc tctgcgaatg catgtataat ttctacagaa      60

cctattagaa aggatcaccc agcctctgct tttgtacaac tttcccttaa aaaactgcca     120

attccactgc tgtttggccc aatagtgaga actttttcct gctgcctctt ggtgcttttg     180

cctatggccc ctattctgcc tgctgaagac actcttgcca gcatggactt aaacccctcc     240

agctctgaca atcctctttc tcttttgttt tacatgaagg gtctggcagc caaagcaatc     300

actcaaagtt caaaccttat cattttttgc tttgttcctc ttggccttgg ttttgtacat     360

cagctttgaa aataccatcc cagggttaat gctggggtta atttataact aagagtgctc     420

tagttttgca atacaggaca tgctataaaa atggaaagat                           460


<210>  35
<211>  488

<212>  DNA
<213>  Homo sapiens

<220>
<221>  misc_feature
<222>  (1)..(488)
<223>  Human SEPP1 promoter region (liver specific promoter)

<400>  35
agcttctctc tctctttttt tttttttttt aaagcagcag cataattcgc tggctcagca      60

aatgtttttc cctgttatag tttgggaaag aaggcaactt ggaggccttg tttaccctta     120

gcccttcctc ccgcccccga ctgtaccatt aaatcctaga ttctagggtg actgaaaagg     180

atagggaata aattagtaat tgtcaaaatg ttttccccat attatgcatg gcagcacttg     240

ctactttctt ttaagttgat aacaatcagc tcaggggttt gctctgcttg caaggtcact     300

gcaagaatga acattgaact ttggactata cctgaggggg tgaggtaaac aacaggacta     360

taaatatcag agtgtgctgc tgtggctttg tggagctgcc agagtaaagc aaagagaaag     420

gaagcaggcc cgttggaagt ggttgtgtaa gtaggaactt cgtgttttct gatactttat     480

tcttttaa                                                              488

<210>  36
<211>  267
<212>  DNA
<213>  Homo sapiens

<220>
<221>  misc_feature
<222>  (1)..(267)
<223>  Human TIE2 enhancer (endothelial cell specific enhancer)

<400>  36
ctaatgtggt atgagtgtga caaagagagg gagaaaattt ggactagcca aagaagccag      60

tcaggcgtgg ggtttgaagg gcatcgtggg cggctgtcat ttgctctctg cttgtcacag     120

ccccttgccc agggcttgac cagtgaggtg tatgtgctgg tcacacccat ctcagcagat     180

ctgtcagctt tcccgctttt gttaaagggt gatatcatgc ttcctggggg gagcactgga     240

agacaatgct cggccacttt cctccag                                         267